(19) Europäisches Patentamt
European Patent Office
Office européen des brevets

(11) EP 2 653 152 A1

(12) **EUROPEAN PATENT APPLICATION**

(43) Date of publication:
**23.10.2013 Bulletin 2013/43**

(21) Application number: **13176687.5**

(22) Date of filing: **29.06.2011**

(51) Int Cl.:
*A61K 9/00* (2006.01)
*A61K 9/12* (2006.01)
*A61P 25/36* (2006.01)
*A61P 25/04* (2006.01)
*A61K 31/4468* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **30.06.2010 GB 201011028**

(62) Document number(s) of the earlier application(s) in accordance with Art. 76 EPC:
**11729456.1 / 2 588 094**

(71) Applicant: **Londonpharma Ltd.**
**Norwich NR4 7UH (GB)**

(72) Inventors:
- **Sams, Martin James**
**Norwich, Norfolk NR4 7UH (GB)**
- **High, Juliet Victoria**
**Norwich, Norfolk NR4 7UH (GB)**
- **Jamieson, Paul Andrew**
**Norwich, Norfolk NR4 7UH (GB)**
- **Ross, Calvin John (Deceased)**
**Norwich, Norfolk NR4 7UH (GB)**

(74) Representative: **Harris, Oliver John Richard**
**Novagraaf UK**
**12 Meridian Way**
**Meridian Business Park**
**Norwich NR7 0TA (GB)**

Remarks:
This application was filed on 16-07-2013 as a divisional application to the application mentioned under INID code 62.

(54) **Pharmaceutical compositions for sublingual delivery of opioids**

(57) The invention provides pharmaceutical compositions for the sublingual delivery of opioids. The invention also provides delivery devices adapted for sublingual delivery of such compositions.

**Figure 1**

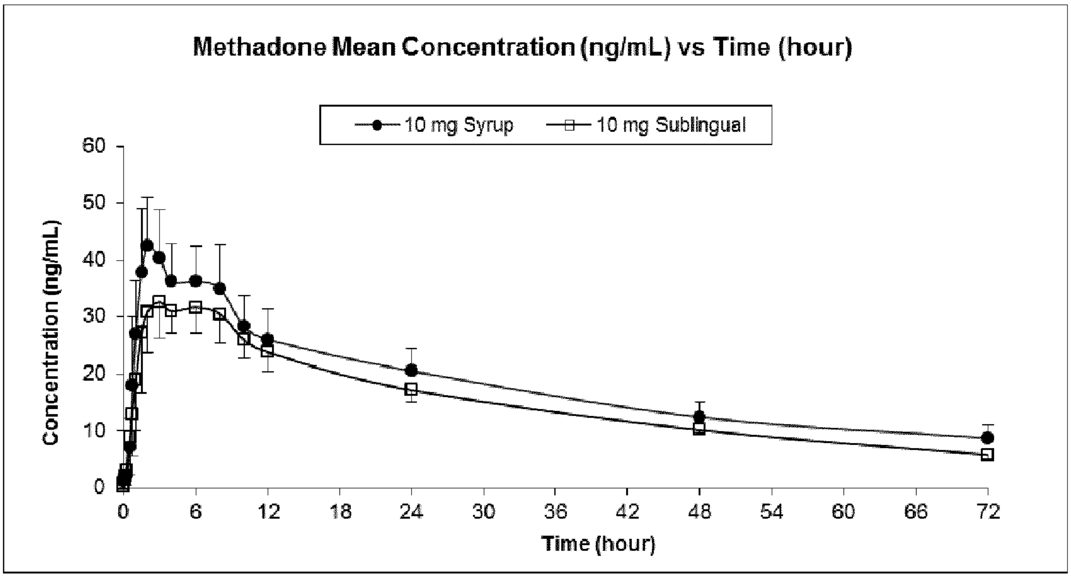


EP 2 653 152 A1

## Description

### Field of the Invention

The invention relates to improved methods of delivery of opioids, and to devices for said delivery.

### Background

The development of drug delivery routes remains an important element in the progress of the pharmaceutical sciences. Once an active compound has been identified, the design of delivery mechanisms must overcome challenges of transporting the medicament to the required site of action in the body whilst addressing issues including shelf stability, bioavailability, toxicity, and patient compliance. All of these challenges must be overcome to achieve the desired therapeutic effect. Amongst the drug delivery options, oral administration is by far the most common route, with other options including injection, topical, inhalation and transmucosal administration.

The oral delivery route faces perhaps the most challenging route for a pharmaceutical to reach the final site of action: the composition is prone to loss from the mouth or stomach (e.g. by spitting or vomiting); the composition must survive the acidic and enzymatically-active environment of the stomach; if not absorbed in the stomach, the medicament must survive the action of bile salts and further intestinal and bacterial enzymatic action within the intestinal tract, be able to cross from the lumen of the gut to the intestinal wall for absorption, and then survive the degradation processes of the liver following transport by the hepatic portal system, often resulting in poor availability due to the first pass effect. Furthermore, many bioactive compounds elicit autoinduction of enzymes (e.g. in the hepatic system) that lead to increasing breakdown of drugs before they reach the systemic circulation, leading to a decrease of bioavailability of the molecules over time during a medicament administration regime. Despite these challenges, the oral route of drug administration remains the most common.

The shelf-stability of a medicament is an important consideration in terms of safety, efficacy and cost. Some medicaments are not stable in traditional delivery devices that are made of e.g. glass or stainless steel (rigid materials required to maintain the shape of the device). Instead, these medicaments are kept in separate, plastic storage containers, with thin, flexible walls making them unsuitable for a delivery device, prior to transfer into a delivery device prior to administration. Such transfer reduces the efficiency of medicament supply: two different containers are required instead of one, and the transfer step introduces the potential for waste and may need to be effected/overseen by a suitable professional (e.g. a pharmacist). The transfer step also introduces the possibility of dispensing error.

Some opioids, such as methadone, are used to treat opioid dependence (as so-called "anti-addictive" drugs). The current methods used to dispense opioids for this purpose are inefficient, particularly for the anti-addictive of choice, methadone. Methadone is usually made up in a glucose syrup suitable (only) for oral administration and stored in bulk (multiple doses) in a lightweight plastic (e.g. polypropylene) bottle. The administration of single doses to the patient requires professional supervision and skill, and includes the accurate measurement of a dispensed single dose and inspection of the patient after dosing to ensure that they have swallowed the dose (some addicts attempt to spit out the dose to then inject it). Other disadvantages of using oral methadone for treating opioid dependence include the potential for contamination of the bulk syrup and the relatively high rate of vomiting in patients who are opioid dependent.

It is among the objectives of the present invention to attempt a solution to these problems.

### Summary of the Invention

The sublingual delivery route in principle offers (for many medicaments, including opioids) substantial benefits over other administration routes. It is particularly beneficial over the oral route in which a medicament is often lost from the mouth or stomach (e.g. spitting or vomiting), degraded by the various enzymatic and other processes in action in the gut, and leads to absorption by the hepatic route, which can lead to significant malabsorption as a result of the "first pass effect" in the liver. As a result, orally-dosed medicaments are often given in greater concentration that would be required if they were well-absorbed and could escape the first-pass effect (often giving rise to unwanted side-effects). Sublingual delivery is also beneficial over the injection route because it provides the possibility of administration by non-medically qualified personnel and avoids the risks associated with injecting.

The inventors have surprisingly identified formulation conditions in which pharmaceutical compositions comprising opioids can be prepared for sublingual delivery. Furthermore, the inventors have identified materials that are, surprisingly, suitable for a container for both the storage and delivery of these compositions.

Accordingly, the invention provides a pharmaceutical composition for the sublingual delivery of an opioid comprising an opioid and ethanol. In preferred embodiments said composition additionally comprises glycerol. In particularly preferred embodiments the opioid is not fentanyl. In further preferred embodiments the opioid is methadone.

Also provided by the invention is any composition as described above that is comprised within a container and

wherein the material of the container that is in contact with the composition is Cyclic Olefin Copolymer (COC).

In an alternative embodiment the invention provides a pharmaceutical composition for the sublingual delivery of an opioid comprising an opioid and a medium chain length triglyceride wherein the composition is comprised within a container and wherein the material of the container that is in contact with the composition is polypropylene. In particularly preferred embodiments the opioid is not methadone. In further preferred embodiments the opioid is fentanyl.

Furthermore, the inventors provide any composition of the invention within a container wherein the container comprises a delivery device. In any aspect of the invention it is particularly preferred that the delivery device dispenses the composition in a single discharge and/or dispenses the composition as a spray. It is preferred that such a spray comprises liquid droplets having a mean diameter of at least about 10 microns, preferably at least about 20 microns, more preferably between about 20 microns and about 200 microns, and most preferably between about 20 microns and about 100 microns.

In any aspect of the invention it is particularly preferred that the delivery device is non-pressurised and/or comprises seals and/or plungers and wherein the material of said seals and/or plungers that is in contact with the composition is bromobutyl polymer.

The inventors also provide a composition of the invention for use in a method of treatment of the human or animal body by therapy, such as for use in a method of:

(a) reducing pain;
(b) inducing or maintaining anaesthesia;
(c) treating opioid dependence;
(d) treating anxiety;
(d) treating a cough; or
(e) treating diarrhoea.

The inventors also provide a method of treating a human or animal subject in need of an opioid comprising the administration to said subject of a therapeutically effective amount of a composition of the invention by the sublingual route, such as wherein said subject:

(a) is suffering from pain, opioid dependence, anxiety, cough or diarrhoea; or
(b) is in need of anaesthesia.

Detailed Description of the Invention

An opioid is a chemical that binds to opioid receptors. Opioids may be broadly classed into natural opioids (the "opiates", alkaloids obtained from the opium poppy), endogenous opioids, semi-synthetic opioids, fully synthetic opioids, and other opioid receptor agonists. Examples of each class are given below:

Natural opioids - morphine, codeine, thebaine and oripavine.

Endogenous opioids - endorphins, enkephalins, dynorphins and endomorphins.

Semi- synthetic opioids- hydromorphone, hydrocodone, oxycodone, oxymorphone, desomorphine, diacetylmorphine (heroin), dihydrocodeine, nicomorphine, dipropanoylmorphine, benzylmorphine, ethylmorphine and buprenorphine.

Fully synthetic opioids - anilidopiperidines (e.g. fentanyl, alphamethylfentanyl, alfentanil, sufentanil, remifentanil, carfentanyl, ohmefentanyl), phenylpiperidines (e.g pethidine, ketobemidone, MPPP, allylprodine, prodine, PEPAP), diphenylpropylamine derivatives (e.g. propoxyphene, dextropropoxyphene, dextromoramide, bezitramide, piritramide, methadone, dipipanone, Levomethadyl Acetate [LAAM], difenoxin, diphenoxylate, loperamide), benzomorphan derivatives (e.g. dezocine, pentazocine, phenazocine), oripavine derivatives (e.g. buprenorphine, dihydroetorphine, etorphine), and morphinan derivatives (e.g. butorphanol, nalbuphine, levorphanol, levomethorphan).

Other opiod receptor agonists - lefetamine, meptazinol, tilidine, tramadol and tapentadol.

Opioids that are particularly envisaged in the invention include methadone, sufentanil and fentanyl, and pharmaceutically acceptable salts thereof, analogues thereof or derivatives thereof. Other opioids envisaged include: alfentanil, buprenorphine, butorphanol, codeine, hydrocodone, hydromorphone, levorphanol, meperidine, morphine, nalbuphine, oxycodone, oxymorphbne, propoxyphene, tramadol, fenpipramide, pentazocine, piritramide, tilidine, tramadol, pharmaceutically acceptable salts thereof, or derivatives thereof, and the like.

In order to avoid oral absorption, the opioid is delivered in a small volume, large enough to coat the sublingual mucosa but small enough to reduce the likelihood that any composition may be swallowed. The skilled addressee will be readily able to determine whether a chosen opioid has sufficient solubility.

Preferably, the opioid is in solution at a concentration providing a required dose of medicament in a volume of no more than 1000microlitres of composition, more preferably in a volume of no more than 500microlitres, more preferably

in a volume of no more than 400 or 300microlitres of composition, more preferably in a volume of no more than 200microlitres of composition, and most preferably in a volume of no more than 100microlitres of composition.

A further preferred feature is that the opioid is stable in the composition, both with respect to physicochemical aspects such as remaining in solution and in terms of chemical (including biochemical) degradation of the medicament over time. It is particularly preferred, therefore, that the opioid is stable within the composition, to pharmaceutically-acceptable limits, over a period of at least one month, preferably at least 2 months, more preferably at least 3 months, more preferably at least 6 months, more preferably at least 12 months, more preferably at least 18 months, more preferably at least 2 years, more preferably at least 3 years, more preferably at least 4 years, and most preferably at least 5 years, whilst kept at a temperature(s) between 4°C and 40°C.

It is also preferred that the opioid is stable in the composition (as defined above) when placed in a container, preferably wherein the container comprises a delivery device, and it is particularly preferred that the opioid is stable within the composition in said container, to pharmaceutically-acceptable limits, over a period of at least one month, preferably at least 2 months, more preferably at least 3 months, more preferably at least 6 months, more preferably at least 12 months, more preferably at least 18 months, more preferably at least 2 years, more preferably at least 3 years, more preferably at least 4 years, and most preferably at least 5 years.

*Formulations comprising ethanol*

In one embodiment the pharmaceutical composition of the invention comprises an opioid and ethanol. In a particular embodiment the pharmaceutical composition of the invention consists essentially of an opioid and ethanol. Preferably ethanol is used in the composition at a concentration of at least 5% (w/w), at least 10% (w/w), at least 15% or at least 18% (w/w) and up to 30% (w/w), up to 40% (w/w) or up to 50% (w/w). Preferably, ethanol is used at a concentration of between 10% (w/w) and 30% (w/w), more preferably at a concentration of between 15% (w/w) and 25% (w/w), such as at a concentration of 18% (w/w) to 22% (w/w). As well as acting as a cosolvent, when ethanol is used at a concentration of more than 18% it also has a preservative effect. Ethanol is particularly useful for sublingual delivery because it evaporates after administration, maintaining the medicament in place on the mucosa.

In a further embodiment the pharmaceutical composition of the invention comprising an opioid and ethanol additionally comprises glycerol. In a particular embodiment the pharmaceutical composition of the invention consists essentially of an opioid, ethanol and glycerol. Preferably glycerol is used in the composition at a concentration of at least 5% (w/w), at least 10% (w/w) or at least 15 % (w/w) and up to 35% (w/w), up to 40% (w/w) or up to 50% (w/w). Preferably, glycerol is used at a concentration of between 15% (w/w) and 35% (w/w), more preferably at a concentration of between 20% (w/w) and 30% (w/w), such as at a concentration of 24% (w/w) or 25% (w/w). Glycerol acts as a sweetener and humectant (moisturiser) and, surprisingly, gives improved opioid solubility and stability (particularly for methadone) in comparison to other traditional humectants (e.g. propylene glycol).

In a further embodiment the pharmaceutical composition of the invention comprising an opioid and ethanol additionally comprises water. In a particular embodiment the pharmaceutical composition of the invention consists essentially of an opioid, ethanol and water. In a further embodiment the pharmaceutical composition of the invention comprising an opioid and ethanol additionally comprises glycerol and water. In a particular embodiment the pharmaceutical composition of the invention consists essentially of an opioid, ethanol, glycerol and water.

In a preferred embodiment the opioid of the composition comprising ethanol is not fentanyl. In a further embodiment the opioid of the composition comprising ethanol is methadone.

If methadone is selected then a total dose is preferably chosen from at least 1mg, at least 5mg, at least 10 mg or at least 15mg and up to 40mg, up to 50mg, up to 60mg, or up to 120mg, preferably between 10mg and 60mg. Particularly preferred total doses include 1mg, 5mg, 10mg, 20mg, 30mg and 60mg. The concentration of methadone selected is preferably at least 10mg/ml, at least15mg/ml, at least 20mg/ml, at least 25mg/ml, at least 50mg/ml or at least 75mg/ml, and up to 100mg/ml, up to 150mg/ml or up to 200mg/ml. Preferably the concentration of methadone selected is between 25mg/ml and 150mg/ml, even more preferably between 25mg/ml and 100mg/ml.

Methadone can be administered from a single dose product (e.g. spray unit) dispensing e.g. a 1mg, 10mg, 20mg, 20mg or 60mg single dose using e.g. a 400μl pump (especially suitable for treating opioid dependence). Methadone can also be administered from a multidose product (e.g. spray unit) dispensing e.g. 30 to 50 lots of e.g. 1mg, 5mg or 10mg doses using e.g. a 100μl pump (especially suitable for reducing pain).

If methadone is selected it may be present as a racemate (e.g. methadone HCl or methadone sulphate) or as the laevorotary or dextrorotary form. L-methadone is particularly suitable for treating opioid dependence, and D-methadone is particularly suitable for reducing pain.

The pharmaceutical composition of the invention comprising an opioid and ethanol is preferably contained within a container (for storage and/or delivery) wherein the material of the container that is in contact with the composition is Cyclic Olefin Copolymer (COC). COC is an amorphous and transparent polymer comprising copolymers based on cycloolefins and linear olefins. The general formula for COC is as follows:

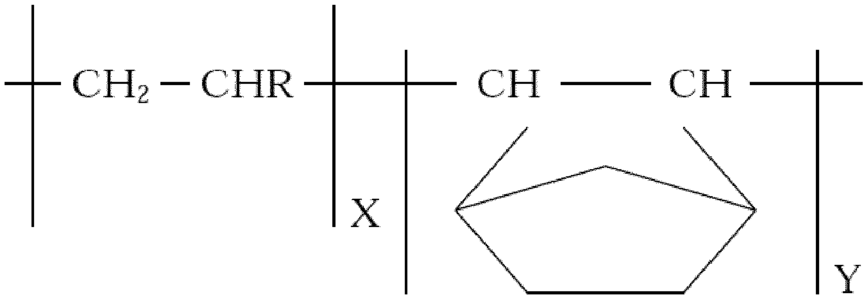

The properties of COC may be varied depending on the exact chemical structure of the copolymer, but typically COC displays low density, high transparency, low birefringence, very low water absorption, excellent water vapour barrier properties, heat deflection temperature up to 170°C, and high rigidity/strength/hardness. These properties make COC a suitable material for medical storage and delivery devices. One suitable source of COC is from the provider Ticona, who market COC under the registered trademark "Topas" (Thermoplastic Olefin Polymer of Amorphous Structure (COC)).

The inventors have surprisingly revealed that some opioid/ethanol compositions (e.g. comprising methadone HCl) are corrosive within containers consisting of glass or stainless steel (materials traditionally used for delivery devices). However, the inventors have identified COC as a suitable material with which opioid/ethanol compositions are compatible, that is to say that such compositions show high stability within containers wherein said compositions are in contact with COC.

The provision of a composition for sublingual delivery of an opioid is advantageous because, in comparison to compositions for oral delivery, it avoids the need for the administrator to ensure that the composition has been swallowed and avoids the bioavailability problems associated with oral delivery (e.g. vomiting).

In relation to methadone, the inventors have surprisingly found that sublingual delivery using a composition of the invention leads to effective uptake without a substantial initial concentration spike, in contrast to the spikes that have previously been seen in the art when other opioids have been administered sublingually (e.g. fentanyl and its derivatives such as sufentanil). The lack of such a methadone concentration spike ensures that this particular embodiment of the composition of the invention is suitable e.g. as a medicament for both treating opioid dependence and reducing pain, because the risk of methadone-induced respiratory depression is significantly reduced. In addition, the lack of a methadone concentration spike ensures that said embodiment does not lead to a significant euphoric effect making it particularly suitable for treating opioid dependence because it is then less likely to be diverted.

The provision of such a composition in a COC container that is suitable for both the storage and delivery of the composition is advantageous because it can provide the administrator with a store of single doses each of which can rapidly be employed to dispense a single dose to a patient without the need for accurate measurement of a dispensed single dose. This arrangement also avoids the risk of contamination associated with bulk (multidose) stores of the opioid and can reduce the potential waste associated with having separate storage/delivery containers.

*Formulations comprising a medium chain length triglyceride*

In an alternative embodiment the pharmaceutical composition of the invention comprises an opioid and a medium chain length triglyceride. In a particular embodiment the pharmaceutical composition of the invention consists essentially of an opioid and a medium chain length triglyceride.

Medium chain length triglycerides are defined in the European Pharmacopoeia Monograph 0868, as:

A mixture of triglycerides of saturated fatty acids, mainly of caprylic acid (octanoic acid, $C_8H_{16}O_2$) and of capric acid (decanoic acid, $C_{10}H_{20}O_2$). Medium-chain triglycerides are obtained from the oil extracted from the hard, dried fraction of the endosperm of *Cocos nucifera* L. or from the dried endosperm of *Elaeis guineensis* Jacq. When Medium-chain Triglycerides are prepared from the endosperm of *Cocos nucifera* L., the title Fractionated Coconut Oil may be used. Medium chain length triglycerides have a minimum 95.0 per cent of saturated fatty acids with 8 and 10 carbon atoms. Further chemical and physical properties are described in the European Pharmacopoeia Monograph 0868, and equivalent documents.

In especially preferred compositions, the triglyceride comprises a minimum of 95 per cent of saturated fatty acids with between 6 and 12 carbon atoms. More preferably, said triglyceride comprises a minimum of 95 per cent of saturated fatty acids with between 8 and 10 carbon atoms.

In a preferred embodiment the triglyceride of the composition is a triglyceride sold under the registered trade mark Miglyol®, and especially a miglyol selected from the group comprising: miglyol 810; miglyol 812; miglyol 818; miglyol 829; and miglyol 840. Preferably the chosen miglyol is miglyol 810. Miglyol® is a medium chain triglyceride containing saturated C8 and C10 fatty acids, typically between 65-80% of caprylic acid (C8:0) and 20-35% of capric acid

(C10:0).

Preferably, the triglyceride constitutes at least 90% (w/w) of the pharmaceutical composition, preferably at least 95% (w/w), more preferably at least 97% (w/w), most preferably at least 98% (w/w) .

In a preferred embodiment the opioid of the composition comprising a medium chain length triglyceride is not methadone. In a further embodiment the opioid of the composition comprising a medium chain length triglyceride is fentanyl.

If fentanyl is selected then a total dose is preferably chosen from at least 50micrograms, at least 100micrograms or at least 200micrograms and up to 500micrograms, up to 800 micrograms , up to 1mg or up to 5mg. Particularly preferred total doses include 200 micrograms and 800 micrograms. The concentration of fentanyl selected is preferably at least 0.01% (w/w), at least 0.05% (w/w) or at least 0.1 % (w/w), and up to 0.2% (w/w), up to 0.5% (w/w) or up to 1% (w/w) . Preferably the concentration of fentanyl selected is between 0.05% (w/w) and 0.5% (w/w) .

In a further embodiment the opioid of the composition comprising a medium chain length triglyceride is an analogue of fentanyl, such as alfentanil, sufentanil, remifentanil, carfentanil and lofentanil.

The pharmaceutical composition of the invention comprising an opioid and a medium chain length triglyceride is preferably contained within a container (for storage and/or delivery) wherein the material of the container that is in contact with the composition is polypropylene (PP). PP is a partially crystalline and transparent polymer. The general formula for PP is as follows:

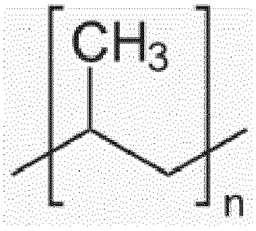

The heat and chemical resistance properties of PP and its rigidity make it a suitable material for medical storage and delivery devices. A suitable source of PP is from the provider Borealis, who market PP under the registered trademark "Bormed" (e.g. Bormed HD810MO).

The inventors have surprisingly revealed that some opioid/triglyceride compositions (e.g. comprising fentanyl and miglyol) are not compatible with COC or indeed with Zylar ® (Styrene Methyl Methacrylate Acrylic copolymer). However, the inventors have identified PP as an alternative suitable material with which opioid/ triglyceride compositions are compatible, that is to say that such compositions show high stability within containers wherein said compositions are in contact with PP.

*Further optional components*

In preferred embodiments any of said compositions, the compositions further comprise a preservative (e.g. propyl or butyl parabens) and/or a flavouring (e.g. blackcurrant flavouring) and/or a sweetener (e.g. sodium saccharin) and/or an essential oil such as menthol, vanillin or orange oil, lemon oil, clove oil, peppermint oil, spearmint oil. The inventors have found that the addition of such an essential oil surprisingly has three benefits: (1) the essential oil acts as a penetration enhancer, improving the rate and extent of uptake of medicaments by the sublingual mucosa; (2) the essential oil, in many cases, acts as a co-solvents thereby increasing the solubility of medicaments; and (3) the essential oil provides a flavour component, giving organoleptic feedback to a user of the medicament, to confirm that is has been successfully delivered.

*Delivery*

Preferably the compositions of the present invention are comprised within a container that comprises a delivery device, and preferably the device dispenses the composition as a single discharge. Preferably the device is non-pressurised.

The compositions of the present invention can be delivered as a liquid bolus or, preferably, as a spray comprising liquid droplets having a mean diameter of at least about 10 microns, preferably at least 20 microns, more preferably from about 20 to about 200 microns, most preferably from about 20 to about 100 microns. Preferably the compositions are delivered as liquid droplets that have a size distribution of from about 5 microns to about 500 microns, preferably from about 10 microns to about 200 microns, more preferably from about 20 microns to about 100 microns. Choice of these droplet sizes ensures that the spray is prevented from passing into the lungs.

Larger droplets have larger weight and this is preferable in the invention because a larger weight increases the chances that the droplet, and therefore the opioid, falls rapidly onto the sublingual mucosa thereby reducing the possibility that the droplets become entrained in breath and expelled from the mouth, or taken into the lungs. It is therefore preferred that, for compositions of the invention comprising ethanol, the weight of a spray droplet is at least 0.4ng, more preferably at least 3.3ng, more preferably at least 400ng, more preferably at least 3.3μg, more preferably at least 5μg. For compositions of the invention comprising miglyol it is preferred that the weight of a spray droplet is at least 0.52ng, more preferably at least 4.2ng, more preferably at least 520ng, more preferably at least 4.2μg, more preferably at least 5μg.

It is particularly preferred that each individual or successive dose has a volume of less than 1000 micro litres. The use of small dose volumes reduces the likelihood that the composition will be swallowed, or spat out, by the patient. The likelihood is reduced further by use of smaller volumes (especially in the paediatric context) and so in further preferred embodiments, each dose has a volume of less than 600 microlitres; less than 500 microlitres; less than 400 microlitres; less than 300 microlitres; less than 200 microlitres; or even less than 100 microlitres. Smaller volumes are especially preferred for paediatric use.

Preferably, the delivery devices according to these aspects comprise a spray, preferably a non- pressurised spray, and especially a pump spray. The use of a pump spray increases the area of mucosa to which the composition is applied, thereby increasing absorption and minimising the likelihood that the medicament is swallowed.

The material of the container/delivery device that makes contact with a composition of the invention should be COC (for compositions comprising ethanol) or PP (for compositions comprising a medium chain length triglyceride). The container/device may also comprise parts that must be elastomeric, such as seals and/or plungers, and for such parts the inventors have identified bromobutyl polymer, such as bromobutyl rubber (a brominated copolymer of isobutylene and isoprene), as a suitable material that is compatible with any composition of the invention (and particularly as a material suitable for making contact with the a composition of the invention).

A suitable source of bromobutyl polymer is from the provider West Pharmaceutical Services, and in particular West Formulation 4023/50 Gray.

*Methods of treatment*

A composition of the invention may be used in a method of treatment of the human or animal body by therapy. In particular, a composition of the invention may be used in a method whereby the application of an opioid confers medical benefit, including methods of:

(a) reducing pain;
(b) inducing or maintaining anaesthesia;
(c) treating opioid dependence;
(d) treating anxiety;
(d) treating a cough; or
(e) treating diarrhoea;

preferably wherein said composition is administered sublingually in said method.

Furthermore, the inventors provide the use of a composition of the invention in the manufacture of a medicament for reducing pain, inducing or maintaining anaesthesia, or treating opioid dependence, anxiety, cough or diarrhoea.

The inventors also provide a method of treating a human or animal subject in need of an opioid comprising the administration to said subject of a therapeutically effective amount of a composition of the invention, whereby administration is by the sublingual route. In such a method the subject may, for example, be suffering from pain, opioid dependence, anxiety, cough or diarrhoea, or may require anaesthesia.

<u>Examples</u>

Example 1 - methadone formulation

**Active Pharmaceutical Ingredient**

The API is supplied by;

Macfarlane Smith
A Johnson Matthey PLC Business
Wheatfield Road
Edinburgh

EH11 2QA
Scotland

An EDMF is available. Methadone hydrochloride is monographed in the Ph Eur, BP and USP. The Ph Eur/BP monograph is given in Appendix I

Its outline properties are;

*(Ph Eur monograph 0408)*

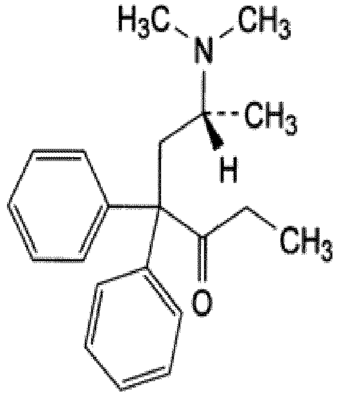

and enantiomer , HCl

$C_{21}H_{27}NO,HCl$ 345.9 1095-90-5

A white, crystalline powder, soluble in water, freely soluble in alcohol.

**Formulation Summary**

Note that the use of the term pH herein covers not only aqueous solutions but also ethanolic aqueous, purely ethanolic and other non-aqueous solutions. Thus the term also covers "apparent pH" as defined in the USP ie the apparent pH reading from formulations not wholly aqueous.

Initial formulation work considered the solubility of methadone hydrochloride at a concentration of 100 mg/ml in aqueous solutions with ethanol and propylene glycol as co-solvents. Dissolution was noticeably faster in solutions containing ethanol as a cosolvent. It was also observed that after storage for approximately 1 month at 4°C and 40°C a fine particulate precipitate was formed in a purely aqueous solution compared with an aqueous ethanolic solution. In addition, methadone dropped out of the propylene glycol based solution after 1 week at 5°C and after 19 weeks at all tested temperatures (in contrast to glycerol based compositions where methadone remained in solution under the same conditions).

It was proposed that to aid sublingual absorption of basic drugs such as methadone the pH should be buffered to approach the pKa of the drug, 8.2 for methadone. Therefore various buffer systems were employed to adjust the pH of the formulation. It was generally observed that when attempts were made to adjust the pH above 7.0 precipitation occurred on mixing or on storage. The precipitate is thought to be methadone base. Therefore it appeared that the use of buffering agent with methadone at pHs over 7 was not possible.

It was decided to concentrate on aqueous formulations containing ethanol (to aid solubility and act as a preservative), propylene glycol or glycerol (moisturiser), sodium saccharin (sweetener) and blackcurrant (flavour) . Several different strengths may be required for the eventual product formulations, therefore to bracket the possible doses required, strengths of 10 mg per 400μl dose (25 mg/ml) and 60 mg per 400μl dose (150 mg/ml) were chosen.

These formulations all proved to be stable over the six month study with no evidence of degradation. However a number of units were observed to have leaked, particularly those at the higher strength. Additionally the contents of a small number of units were observed to have changed colour to orange brown. These were observed at all temperatures and across the formulations and also in placebo units that had been prepared. A new ultrasonic welder was found to give a much improved and consistent seal.

It was therefore decided to repeat the formulations containing glycerol (instead of propylene glycol) but omitting

the blackcurrant flavour. These formulations proved to be stable after 6 months storage and were chosen for progression to a Phase I study as 10, 20 & 30 mg formulations. These lots were prepared to GMP, placed on stability and one and three month data was satisfactory.

**Formulation**

Following initial preformulation work the following formulations were prepared at 100mg ml$^{-1}$ (50ml);

| | PD01/07 | | PD01/08a | | PD01/08b | |
|---|---|---|---|---|---|---|
| | g | % w/w | g | % w/w | g | % w/w |
| Methadone HCl | 5.00 | 9.03 | 5.00 | 9.47 | 5.00 | 8.95 |
| Sodium Saccharin | 0.40 | 0.72 | 0.40 | 0.76 | 0.40 | 0.72 |
| Ethanol, anhydrous | | | 9.88 | 18.71 | | |
| Propylene Glycol | | | | | 12.95 | 23.19 |
| Purified Water | 50.0 | 90.25 | 37.50 | 71.06 | 37.50 | 67.14 |
| | 55.40 | 100.00 | 52.78 | 100.00 | 55.85 | 100.00 |
| | | | | | | |

1. 50mg methadone hydrochloride was weighed into a 50ml volumetric flask.
2. Approximately 35mls of solvent was added and the flask shaken until the methadone dissolved.
3. Sodium saccharin was added to the flask and shaken until fully dissolved.
4. The flask was made up to volume with solvent and shaken until homogeneous.
5. The pH was adjusted to 8.2 with 0.1M NaOH.

PD01/07 required ultrasonication to dissolve the methadone HCL. Na saccharin dissolved readily. Initial pH 4.7. Much 0.1M NaOH was added with precipitation at each addition which re-dissolved. Final pH 7.0.

PD01/08a dissolved the API on shaking as well as the Na saccharin. Initial pH 4.9, adjustment as the previous formulation.

PD01/08b required ultrasonication to dissolve the methadone HCL. Na saccharin dissolved readily. Initial pH 4.9, adjustment as the previous formulations.

None of these initial formulations could be raised to a higher pH than 7.0 due to precipitation. Each formulation was transferred into serum bottles and placed on storage at 4, 25 and 40°C.

After storage for 1 week all samples remained clear, colourless solutions except for PD01/08b (comprising propylene glycol) at 4°C which had significant precipitation. The solutions were allowed to equilibrate to room temperature and examined after six months storage;

PD01/07; all solutions were clear and colourless with fine white crystals at 4°C and needle-like white crystals at 25°C. No particulates at 40°C. The samples were not re-examined.

PD01/08a; all solutions were clear and colourless with no particulates. The pH of the solutions was;

4°C 7.2.
25°C 7.1
40°C 7.1

After 14 months storage the 25 and 40°C solutions were unchanged, the 4°C sample was as the other temperatures but with a number of crystals present.

PD01/08b; all solutions were clear and colourless with small white crystals adhering to the glass at 4°C and two large white crystals at 25°C. No particulates at 40°C. The samples were not re-examined.

The above emphasises that ethanolic aqueous formulations are more suitable for methadone formulation.

The level of ethanol in the formulations was explored using the following formulations (50ml);

| | PD01/11a | | PD01/11b | | PD01/11c | |
|---|---|---|---|---|---|---|
| | g | % w/w | g | % w/w | g | % w/w |
| Methadone HCl | 5.00 | 9.2 | 5.00 | 9.3 | 5.00 | 9.4 |
| Sodium Saccharin | 0.40 | 0.7 | 0.40 | 0.7 | 0.40 | 0.7 |
| Ethanol, anhydrous | 3.16 | 5.8 | 5.53 | 10.3 | 7.90 | 14.8 |
| Purified Water | 46.00 | 84.3 | 43.00 | 79.7 | 40.00 | 75.1 |
| | 54.56 | 100.00 | 53.93 | 100.00 | 53.30 | 100.00 |
| 1. The methadone hydrochloride was weighed into a 50ml volumetric flask.<br>2. The ethanol was added followed by water to approximately 30ml.<br>3. The flask was shaken to dissolve the methadone.<br>4. The sodium saccharin was added and dissolved by shaking.<br>5. The volume was made up with water. | | | | | | |

The dissolving of methadone was notably slower in PD01/11a than with the other formulations with higher levels of ethanol. The sodium saccharin dissolved readily in all formulations. The pH of all formulations was 5.0. Each formulation was transferred into serum bottles and placed on storage at 4, 25 and 40°C. After four months storage the solutions were allowed to equilibrate to room temperature and examined;

PD01/11a; 4°C contained a large quantity of white crystalline material, 25 & 40 °C were clear colourless solutions. PD01/11b; As PD01/11a but not so much material at 4°C. PD01/11c; all solutions were clear and colourless, pH;

4°C 5.1
25°C 5.3
40°C 5.3

After 13 months storage PD01/11c 25 and 40°C samples were unchanged, 4°C sample was as the other temperatures with the addition of fine white needle crystals.

The previous formulation work has shown that attempts to raise the pH of the formulations has resulted in the formation of a precipitate which can be redissolved in ethanol, anhydrous. Therefore if a higher pH is required the formulation will need the presence of ethanol to keep the methadone base in solution. The following formulation was prepared using pH 8.5 phosphate buffer;

| | PD01/17 | | |
|---|---|---|---|
| | g | % w/w | Batch |
| Methadone HCl | 6.000 | 10.96 | Macfarlan Smith 06-00988 |
| Ethanol, anhydrous | 5.530 | 10.10 | Hayman 07/101 A2 |
| Propylene Glycol | 5.698 | 10.41 | Merk K37090378 718 |
| Phosphate Buffer | 37.500 | 68.52 | |
| | 54.728 | 99.99 | |
| 8.5 Buffer | | | |
| Na Dihydrogen Phosphate | | 0.6 | BDH A69182 |
| Na Hydroxide | | | VWR Prolabs J005 |
| Purified Water | | To 100 | |

1. The methadone was weighed into a 50ml flask and the ethanol added.
2. The flask was stirred to dissolve the methadone.
3. Stage 2 did not result in a solution so the propylene glycol was added and stirred again without producing a solution.
4. The phosphate buffer was added to within 5 ml of the total volume and mixed.
5. The pH was adjusted to 8.5 and the solution made up to volume.

As the phosphate buffer was gradually added with mixing the methadone dissolved. As more was added a precipitate formed from pH 7.2. The formulation was transferred to a 100ml flask and ethanol added in 10ml portions. After the addition of 50mls of ethanol the precipitate dissolved to give a clear, colourless solution pH 7.2. From the above it appears that the ethanolic aqueous type formulation is incapable of formulation at pH higher than approximately 7.0. The solution was checked after three months storage and found to be clear and colourless with white crystals and so was discarded.

The effect of raising the ethanol level was investigated in the following formulation;

| | PD01/20 | | |
|---|---|---|---|
| | g | % w/w | Batch |
| Methadone HCl | 6.00 | 11.8 | Macfarlan Smith 06-00988 |
| Ethanol, anhydrous | 19.75 | 38.9 | Hayman 07/101 A2 |
| Phosphate Buffer | 25.90 | 49.3 | |
| | 51 .65 | | |

The method of preparation was as above (50ml). The methadone failed to dissolve in the ethanol but initially dissolved on addition of the buffer. As approximately 20ml buffer was added transient precipitation occurred but rapidly cleared with stirring. The final pH was 7.3. The solution was filled into serum bottles and stored at 4°C. After three months storage the solution was found to remain clear, colourless and particle free, pH 7.2. After one year's storage very fine white crystals were observed.

Following from the above formulations were prepared using water and citrate buffer;

| | PD01/22a | | PD01/22b | | Batch |
|---|---|---|---|---|---|
| | g | % w/w | g | % w/w | |
| Methadone HCl | 6.000 | 10.96 | 6.000 | 10.96 | Macfarlan Smith 06-00988 |
| Ethanol, anhydrous | 5.530 | 10.10 | 5.530 | 10.10 | Hayman 07/101 A2 |
| Propylene Glycol | 5.698 | 10.41 | 5.698 | 10.41 | Merk K37090378 718 |
| Purified Water | 37.500 | 68.52 | | | |
| Citrate Buffer | | | 37.500 | 68.52 | |
| | 54.728 | 99.99 | 54.728 | 99.99 | |
| | | | | | |
| Citrate Buffer | | | | | |
| Citric Acid | 2.4 | | | | Fisher 0587121 |
| Sodium Hydroxide | 1.4 | | | | VWR Prolabo J005 |
| Purified Water | To 250ml | | | | |
| | | | | | |
| pH formulation | 4.8 | | 6.7 | | |
| | | | | | |

The buffer was adjusted to pH 7.0 with 1M sodium hydroxide.

For both formulations the methadone hydrochloride dissolved within five minutes in the water/buffer and ethanol mix. The propylene glycol mixed into the solution easily leaving a clear, colourless solution. The solutions were placed at 4°C storage. After storage for up to one month the solutions were examined physically and found not to have changed pH. After 2½ months storage no change was observed and the pHs were 5.2 and 6.9 respectively. After 11 months storage no change was noted.

A formulation review was conducted at this stage in the study. It was decided to concentrate on aqueous formulations containing ethanol (to aid solubility and act as a preservative), propylene glycol or glycerol (moisturiser), sodium saccharin (sweetener) and blackcurrant (flavour) . Several different strengths may be required for the eventual

product formulations, therefore to bracket the possible doses required, strengths of 10 mg per 400μl dose (25 mg/ml) and 60 mg per 400μl dose (150 mg/ml) were chosen and 100ml volumes of each of the following formulations were prepared;

| | PD01/36a | | PD01/36b | | PD01/36c | | PD01/36d | |
|---|---|---|---|---|---|---|---|---|
| | mg unit$^{-1}$ | % w/w | mg unit$^{-1}$ | % w/w | mg unit$^{-1}$ | % w/w | mg unit$^{-1}$ | % w/w |
| Methadone HCl | 60.0 | 13.53 | 60.0 | 12.99 | 10.0 | 2.53 | 10.0 | 2.42 |
| Blackcurrant flavour | 2.2 | 0.50 | 2.2 | 0.48 | 2.0 | 0.51 | 2.0 | 0.48 |
| Na Saccharin | 1.1 | 0.25 | | | 1.0 | 0.25 | | |
| Ethanol, anhydrous | 90.0 | 20.29 | 90.0 | 19.48 | 80.0 | 20.22 | 80.0 | 19.40 |
| Propylene glycol | 110.0 | 24.80 | | | 100.0 | 25.28 | | |
| Glycerol | | | 115.0 | 24.89 | | | 105.0 | 25.46 |
| Water | 180.3 | 40.64 | 194.8 | 42.16 | 202.6 | 51.21 | 215.4 | 52.23 |
| | 443.6 | 100.01 | 462.0 | 100.00 | 395.6 | 100.00 | 412.4 | 99.99 |
| | | | | | | | | |
| Mean fill weight mg | 392.8 | | 414.5 | | 390.8 | | 406.4 | |
| RSD % | 1.3 | | 1.6 | | 1.0 | | 1.0 | |

| | Suppler | Batch |
|---|---|---|
| Methadone HCl | Macfarlan Smith | 06-00988 |
| Blackcurrant flavour | Firmenich | 17577392 |
| Na Saccharin | Merck | S37153 328 |
| Ethanol, anhydrous | Hayman | 07/101 A2 |
| Propylene glycol | Merck | K37090378 718 |
| Glycerol | VWR | 07D120030 |

The formulations were prepared in 100ml volumetric flasks with shaking, all were clear and colourless. Placebo formulations were also prepared for each of the above (lots a & b; mean fill weights 388.5 & 406.2mg, RSD 0.5 & 0.6% respectively). The formulations were filled into 70 spray units (400μl) and the remainder into serum bottles. The samples were stored under ICH conditions at 5, 25/60, 30/65 & 40/75 °C/RH. The spray units were weighed before being placed on storage - see stability sections below for results.

These formulations all proved to be stable over the six month study with no evidence of degradation (see stability sections below for results). However a number of units were observed to have leaked particularly those at the higher strength. The percentage of units that had leaked is shown below. Additionally the contents of a small number of units were observed to have changed colour to orange brown. These were observed at all temperatures and across the formulations and also in placebo units that had been prepared.

| | PD01/036a | PD01/036b | PD01/036c | PD01/036d |
|---|---|---|---|---|
| | 20% | 16% | 4% | 6% |

It was therefore decided to repeat the formulations containing glycerol (as PD01/036b and PD01/036d) but omitting the blackcurrant flavour. 100ml volumes of the following formulations were therefore prepared in 100ml volumetric flasks with shaking.

| | PD01/049 | | PD01/051 | |
|---|---|---|---|---|
| | g | % w/w | g | % w/w |
| **Methadone HCl** | 15.01 | 14.0 | 2.54 | 2.5 |
| **Ethanol, anhydrous** | 20.08 | 18.8 | 20.21 | 19.7 |
| **Glycerol** | 25.00 | 23.4 | 25.08 | 24.4 |
| **Purified water** | 46.96 | 43.9 | 54.98 | 53.5 |
| **TOTAL** | 107.05 | 100.1 | 102.81 | 100.1 |

**Materials**

| | Supplier | Batch |
|---|---|---|
| Methadone hydrochloride | Macfarlan Smith | 06-00988 |
| Ethanol, anhydrous | Hayman | 07/875 A1 |
| Glycerol | VWR | 07D120030 |
| Water | Lab supply | |

For each formulation 100 spray unitdevices were filled with 400μl and placed on stability at ICH 5°C, 25°C/ 60%RH, 40°C/75%RH for a six month period, results are shown in the stability sections below. At two weeks storage the samples were checked for discolouration and weight loss. No discoloration was observed in any sample and the weight loss was satisfactory except for 5 units found to have high weight loss. At the one month timepoint 6/180 units had weight loss > 5% over all storage conditions. No discolouration was observed. At 2 months 10/144 units had weight loss > 5%. No discolouration was observed. At 3 months 8/108 units had weight loss > 5%. No discolouration was observed. At 6months 4/72 had weight loss >5%. No discolouration was observed.

| | PD01/049 | PD01/051 |
|---|---|---|
| **Mean fill weight (mg)** | 418.6 | 410.5 |
| **RSD (%)** | 2.6 | 1.9 |

These formulations proved to be stable and were chosen for progression to a Phase I study as 10, 20 & 30 mg formulations.

In preparation for this, laboratory batches were prepared to check that the formulations deliver the correct dose. As some of the product is retained by the device and based on experience with earlier lots an 8% overage was used. The formulations (10 & 20mg were prepared in 100ml volumetric flasks; 30mg was a 11 scale-up batch) were;

| | PD01/59a | PD01/59b | PD01/59c | PD01/60 | Batch |
|---|---|---|---|---|---|
| | 10mg | 20mg | 30mg | 30mg | |
| Methadone HCl | 2.7g | 5.4g | 8.2g | 81.0g | 06-00988 |
| Ethanol, anhydrous | 20.5g | 20.5g | 20.5g | 205.0g | 07/875 A1 |
| Glycerol | 25.0g | 25.0g | 25.1g | 250.0g | 07D120030 |
| Water | to 100ml | to 100ml | to 100ml | 489.0g | |
| | | | | 1025g | |

The batches were assayed;

| | PD01/59a | PD01/59b | PD01/59c | PD01/60 |
|---|---|---|---|---|
| Methadone HCl mg/ml | 27.6 | 56.4 | 83.9 | 83.3 |

For the clinical trial supplies lots were prepared to GMP in a licensed facility using the ultrasonic welder and placed on stability test. The batches manufactured were;

| | |
|---|---|
| 08-212 | 10mg |
| 08-213 | 20mg |
| 08-214 | 30mg |

| | 08-212 | 08-213 | 08-214 | Batch ESN |
|---|---|---|---|---|
| | 10mg | 20mg | 30mg | |
| Methadone HCl | 27.0g | 54.0g | 81.0g | 4175 |
| Ethanol, anhydrous | 205.0g | 205.0g | 205.0g | 4163 (plus 35.5g 4180 in 08-214) |
| Glycerol | 250.0g | 250.0g | 250.0g | 4162 |
| Water | 538.0g | 516.0g | 486.0g | 4157 |
| | 1020g | 1025g | 1022g | |

The batches were prepared as 1L lots filled at 400μl. 615 units (approximately) were prepared from each lot. No issues were encountered during manufacture. Half the stability samples were packed in heat sealed aluminium pouches. See the stability sections below for the stability results.

**Device Design & Manufacture**

The main body of the device was composed of Topas® COC. A bromobutyl polymer was used for the drug chamber stopper and screw cap stopper (West Pharmaceutical Services, West Formulation 4023/50 Gray).

**Analytical Method Development and Validation - HPLC**

The Ph Eur monograph for methadone hydrochloride does not have a HPLC method. A method was used on the HP1050 system using the following;

| | |
|---|---|
| Column | Phenominex Gemini C18 150 x 4.6mm (HC-COL-001) |
| Flow | 1.0 ml min$^{-1}$ |
| Detector | uv @ 210nm |
| Column Temperature | 30°C |
| Injection Volume | 2μl |
| Mobile Phase | 1:1 v/v acetonitrile:water 0.1% trifluoroacetic acid |

The run time for methadone hydrochloride was found to be 5.1 minutes with one other peak (0.19%) at 2.6 minutes.

It was felt that the use of a milder buffer agent would be preferable so a pH 3.0 phosphate buffer would be evaluated keeping the remaining method details the same but running on the Agilent 1100 system. Initially the methadone hydrochloride peak was at 2.65 minutes but reducing the acetonitrile content to 40% gave 4.85 minutes and to 35% gave 8.5 minutes.

In order to show that degradation of methadone is detected and quantified by the HPLC method the solutions prepared as PD01/01 were taken after 2½ months storage and had reagents added to force degradation. Details of PD01/01 are;

A 100μg/ml methadone hydrochloride in water stored at 4°C
B 100μg/ml methadone hydrochloride in water stored at 40°C
C 100μg/ml methadone hydrochloride in water/ethanol, 50:50, stored at 4°C
D 100μg/ml methadone hydrochloride in water/ethanol, 50:50, stored at 40°C

The solutions were filtered and 4 x 750μl aliquots of each solution were added to 4 separate 1.5ml amber HPLC vials. 750μl of the appropriate reagent was then added as listed below;

1M Hydrochloric Acid - Vial 1
0.1M Sodium Hydroxide - Vial 2
6% v/v Hydrogen Peroxide - Vial 3
Purified Water - Vial 4

A cap was crimped onto the vials and placed at 25°C for a week. The vials to which the sodium hydroxide solution was added turned a milky white. After a week's storage vials from lot A were diluted to 10ml with mobile phase and run on the HP1050 using the above method. Some degradation was noted for the hydrogen peroxide samples; in particular the following peaks;

2.45 mins 0.4%
2.60 mins 1.78% (two peaks)
2.97 mins 0.3%

The solution with sodium hydroxide added gave low methadone assays probably due to insoluble methadone base. The results show methadone to be stable (no detected degradation) with acids and bases. However the degradation found with the addition of hydrogen peroxide shows methadone may be susceptible to oxidation.

**Stability Summary**

PD01/07 (aqueous) and PD01/08b (aqueous/propylene glycol) both with sodium saccharin were examined after 14 weeks storage at 4, 25 & 40°C and found not to have degraded. However after six months storage PD01/07 had white crystals at 4°C and 25°C. PD01/08b had white crystals at 4°C from 1 week onwards and at 25°C at 6 months. PD01/08a (aqueous ethanolic) had clear colourless solutions at 4, 25 and 40°C at 14 months. From this it appears that he best formulation type for the product should be based on aqueous ethanolic solutions (omitting propylene glycol).

PD01/11a, b & c which were aqueous formulations with escalating levels of ethanol were examined after 6 and 13 weeks at 4, 25 & 40°C and found not to have degraded. Physically all formulations gave clear colourless solutions at 4, 25 and 40°C at 13 weeks. After 4 months storage PD01/11a had white crystals at 4°C as did PD01/11b. PD01/11c had no crystal at any temperature. However at 13months PD01/11c had white crystals at 4°C. From this it appears that at least 15% ethanol in an aqueous solution is required to maintain methadone solubility.

PD01/20 was prepared which has a higher (38.9%) level of ethanol and used a phosphate buffer. It was stored at 4°C and was a clear colourless solution at 3 months but had white crystals at 1 year.

Pod01/22 a & b, ethanol/water and ethanol/aqueous citrate buffer respectively formulations with propylene glycol were examined after 4 weeks storage at 4°C and found not to have degraded. Methadone was 11 %. Physically the solutions when stored at 4°C were clear and colourless at 2 weeks, 10 weeks and 11 months.

PD01/36a - d were 60 and 10mg formulations based on aqueous/ethanolic solvent containing blackcurrant flavour with either sodium saccharin or glycerol. The sodium saccharin formulations also contained propylene glycol. The formulations were filled into spray devices as described above. The devices stored at 5, 25, 30 and 40°C ICH conditions were examined after 1, 2 and 6 months and were found not to have degraded. Assay, delivered dose and ethanol content results were all satisfactory. After three months storage some units at 5°C across all formulations and placebos were discoloured pale orange/brown. The stored solutions were clear and colourless. At six months the discolouration was noted again. The discoloration was attributed to the blackcurrant flavour.

The glycerol formulations outlined in the previous paragraph were repeated with the blackcurrant flavour omitted; PD01/049 & 51 (60 and 10mg) and filled into the spray devices. The devices stored at 5, 25, and 40°C ICH conditions were examined after 1, 2 and 6 months and were found not to have degraded. All solutions were clear and colourless. Assay, delivered dose and ethanol content results were all satisfactory.

It was concluded that the discolouration issue had been resolved and that these formulations formed the basis for formulations suitable to be taken into a Phase I study. The study would, for safety reasons, have an escalating dose of 10, 20 and 30 mg only. It was decided that an 8% overage would be applied to the methadone HCl concentration to allow for material left in the device ie the devices would deliver 10, 20 & 30 mg methadone HCl.

The clinical trial batches were prepared in a GMP facility as; 08-212, 08-213 & 08-214 for the 10, 20 & 30mg batches respectively. Samples were placed on stability storage at 5, 25, 30 and 40°C ICH conditions, half the samples were packed in heat sealed aluminium pouches. Units have been examined at 1, 3 and 6 months. No changes in physical appearance have been found at any temperature. Assay, delivered dose, methadone HCl concentration and ethanol content results were all satisfactory. No significant degradation has been observed at any temperature.

Stability at 9months was completed for units from 5, 25 and 30°C ICH conditions and all results for content by HPLC and ethanol content by GC were in limits and no colour change or solubility issues were seen at this time point. Due to contractual problems stability at 12 months was not possible therefore stability at 16 months was completed. The units were tested at 5, 25 and 30°C; all HPLC results were within limits and GC results (with the exception of one sample) were within limits. No colour change or solubility issues were seen at this time point. No degradation was observed and this product has a shelf life of 2 years.

**Stability Results**

Definitions:

Description - conforms if clear, colourless solution

Degradation -

As ICH guidelines;

Reporting threshold = 0.1%

Identification threshold = 0.2%

Qualification threshold = 0.5%

Uniformity of content -

The preparation complies with the test if not more than one individual content is outside the limits of 85 per cent to 115 per cent of the average content and none is outside the limits of 75 per cent to 125 per cent of the average content. The preparation fails to comply with the test if more than three individual contents are outside the limits of 85 per cent to 115 per cent of the average content or if one or more individual contents are outside the limits of 75 per cent to 125 per cent of the average content. If 2 or 3 individual contents are outside the limits of 85 per cent to 115 per cent but within the limits of 75 per cent to 125 per cent, determine the individual contents of another 20 dosage units taken at random. The preparation complies with the test if not more than three of the individual contents of the 30 units are outside the limits of 85 per cent to 115 per cent of the average content and none is outside the limits of 75 per cent to 125 per cent of the average content.

Uniformity of mass -

Determine the individual masses of 10 containers emptied as completely as possible, and calculate the average mass. Not more than 2 of the individual masses deviate by more than 10 per cent from the average mass and none deviates by more than 20 per cent.

**PD01/07 & 08a**

| | PD01/07 | | PD01/08a | | PD01/08b | |
|---|---|---|---|---|---|---|
| | g | % w/w | g | % w/w | g | % w/w |
| Methadone HCl | 5 | 9.03 | 5.00 | 9.47 | 5.00 | 8.95 |
| Sodium Saccharin | 0.4 | 0.72 | 0.40 | 0.76 | 0.40 | 0.72 |
| Ethanol, anhydrous | | | 9.88 | 18.71 | | |
| Propylene Glycol | | | | | 12.95 | 23.19 |
| Purified Water | 50 | 90.25 | 37.50 | 71.06 | 37.50 | 67.14 |
| | 55.4 | 100.00 | 52.78 | 100.00 | 55.85 | 100.00 |
| | | | | | | |

**PD01/07**

| | Assay methadone μg/ml | Appearance of solution | Degradation % | | |
|---|---|---|---|---|---|
| | | | | | |
| | | | | | |
| Initial | | Clear, colourless | | | |
| **4°C** | | | | | |
| 1 week | | Clear, colourless | | | |
| 4 weeks | | Clear, colourless | | | |
| 6 weeks | | Clear, colourless | | | |
| 14 weeks | 93.7 | Clear, colourless | No significant degradation was found | | |
| 6 months | | Clear, colourless plus white crystals | | | |
| | | | | | |
| **25°C** | | | | | |
| 1 week | | Clear, colourless | | | |
| 4 weeks | | Clear, colourless | | | |
| 6 weeks | | Clear, colourless | | | |
| 14 weeks | 93.2 | Clear, colourless | No significant degradation was found | | |
| 6 months | | Clear, colourless plus white crystals | | | |
| | | | | | |
| **40°C** | | | | | |
| 1 week | | Clear, colourless | | | |
| 4 weeks | | Clear, colourless | | | |

(continued)

| | | | | | |
|---|---|---|---|---|---|
| **40°C** | | | | | |
| 6 weeks | | Clear, colourless | | | |
| 14 weeks | 96.0 | Clear, colourless | No significant degradation was found | | |
| 6 months | | Clear, colourless | | | |

**PD01/08a**

| | Assay methadone μg/ml | Appearance of solution | Degradation % | | |
|---|---|---|---|---|---|
| | | | | | |
| | | | | | |
| Initial | | Clear, colourless | | | |
| **4°C** | | | | | |
| 1 week | | Clear, colourless | | | |
| 14 weeks | 94.0 | Clear, colourless | No significant degradation was found | | |
| 6 months | | Clear, colourless | | | |
| 14 months | | Clear, colourless plus white crystals | | | |
| | | | | | |
| **25°C** | | | | | |
| 1 week | | Clear, colourless | | | |
| 14 weeks | 92.8 | Clear, colourless | No significant degradation was found | | |
| 6 months | | Clear, colourless | | | |
| 14 months | | Clear, colourless | | | |
| | | | | | |
| **40°C** | | | | | |
| 1 week | | Clear, colourless | | | |
| 14 weeks | 95.5 | Clear, colourless | No significant degradation was found | | |
| 6 months | | Clear, colourless | | | |
| 14 months | | Clear, colourless | | | |

**PD01/08b**

| | Assay methadone μg/ml | Appearance of solution | Degradation % | | |
|---|---|---|---|---|---|
| | | | | | |
| | | | | | |
| Initial | | Clear, colourless | | | |

(continued)

| | | | | | |
|---|---|---|---|---|---|
| **4°C** | | | | | |
| 1 week | | Clear, colourless plus white crystals | | | |
| 14 weeks | | Clear, colourless plus white crystals | No significant degradation was found | | |
| 6 months | | Clear, colourless plus white crystals | | | |
| | | | | | |
| **25°C** | | | | | |
| 1 week | | Clear, colourless | | | |
| 14 weeks | | Clear, colourless | No significant degradation was found | | |
| 6 months | | Clear, colourless plus white crystals | | | |
| | | | | | |
| **40°C** | | | | | |
| 1 week | | Clear, colourless | | | |
| 14 weeks | | Clear, colourless | No significant degradation was found | | |
| 6 months | | Clear, colourless | | | |

**PD01/11a, b & c**

| | PD01/11a | | PD01/11b | | PD01/11c | |
|---|---|---|---|---|---|---|
| | g | % w/w | g | % w/w | g | % w/w |
| Methadone HCl | 5.00 | 9.2 | 5.00 | 9.3 | 5.00 | 9.4 |
| Sodium Saccharin | 0.40 | 0.7 | 0.40 | 0.7 | 0.40 | 0.7 |
| Ethanol, anhydrous | 3.16 | 5.8 | 5.53 | 10.3 | 7.90 | 14.8 |
| Purified Water | 46.00 | 84.3 | 43.00 | 79.7 | 40.00 | 75.1 |
| | 54.56 | 100.00 | 53.93 | 100.00 | 53.30 | 100.00 |

**PD01/11a**

| | Assay methadone μg/ml | Appearance of solution | Degradation % | | |
|---|---|---|---|---|---|
| Initial | | Clear, colourless | | | |
| **4°C** | | | | | |
| 6 weeks | | Clear, colourless | No significant degradation was found | | |
| 13 weeks | 99.7 | Clear, colourless | No significant degradation was found | | |
| 4 months | | Clear, colourless plus white crystals | | | |

(continued)

| | | | | | |
|---|---|---|---|---|---|
| **4°C** | | | | | |
| | | | | | |
| **25°C** | | | | | |
| 6 weeks | | Clear, colourless | No significant degradation was found | | |
| 4 months | | Clear, colourless | No significant degradation was found | | |
| | | | | | |
| **40°C** | | | | | |
| 6 weeks | | Clear, colourless | No significant degradation was found | | |
| 13 weeks | 100.5 | Clear, colourless | No significant degradation was found | | |
| 4 months | | Clear, colourless | | | |

**PD01/11b**

| | **Assay methadone μg/ml** | **Appearance of solution** | **Degradation %** | | |
|---|---|---|---|---|---|
| Initial | | Clear, colourless | | | |
| **4°C** | | | | | |
| 6 weeks | | Clear, colourless | No significant degradation was found | | |
| 13 weeks | 99.5 | Clear, colourless | No significant degradation was found | | |
| 4 months | | Clear, colourless plus white crystals | | | |
| | | | | | |
| **25°C** | | | | | |
| 6 weeks | | Clear, colourless | No significant degradation was found | | |
| 4 months | | Clear, colourless | | | |
| | | | | | |
| | | | | | |
| **40°C** | | | | | |
| 6 weeks | | Clear, colourless | No significant degradation was found | | |
| 13 weeks | 96.7 | Clear, colourless | No significant degradation was found | | |
| 4 months | | Clear, colourless | | | |

**PD01/11c**

| | **Assay methadone μg/ml** | **Appearance of solution** | **Degradation %** | | |
|---|---|---|---|---|---|
| Initial | | Clear, colourless | | | |
| **4°C** | | | | | |
| 6 weeks | | Clear, colourless | No significant degradation was found | | |

(continued)

| | | | | | |
|---|---|---|---|---|---|
| **4°C** | | | | | |
| 13 weeks | 100.1 | Clear, colourless | No significant degradation was found | | |
| 4 months | | Clear, colourless | | | |
| 13 months | | Clear, colourless plus white crystals | | | |
| | | | | | |
| **25°C** | | | | | |
| 6 weeks | | Clear, colourless | No significant degradation was found | | |
| 4 months | | Clear, colourless | | | |
| 13 months | | Clear, colourless | | | |
| | | | | | |
| | | | | | |
| **40°C** | | | | | |
| 6 weeks | | Clear, colourless | No significant degradation was found | | |
| 13 weeks | 99.4 | Clear, colourless | No significant degradation was found | | |
| 4 months | | Clear, colourless | | | |
| 13 months | | Clear, colourless | | | |

**PD01/22a&b**

| | **PD01/22a** | | **PD01/22b** | |
|---|---|---|---|---|
| | **g** | **% w/w** | **g** | **% w/w** |
| Methadone HCl | 6.000 | 10.96 | 6.000 | 10.96 |
| Ethanol, anhydrous | 5.530 | 10.10 | 5.530 | 10.10 |
| Propylene Glycol | 5.698 | 10.41 | 5.698 | 10.41 |
| Purified Water | 37.500 | 68.52 | | |
| Citrate Buffer | | | 37.500 | 68.52 |
| | 54.728 | 99.99 | 54.728 | 99.99 |
| | | | | |
| Citrate Buffer | | | | |
| Citric Acid | 2.4 | | | |
| Sodium Hydroxide | 1.4 | | | |
| Purified Water | To 250ml | | | |
| | | | | |
| pH formulation | 4.8 | | 6.7 | |
| | | | | |

**PD01/22a**

| | Assay methadone μg/ml | | Appearance of solution | Degradation % | | |
|---|---|---|---|---|---|---|
| | | | | | | |
| | U1 | U2 | | | | |
| Initial | | | Clear, colourless | | | |
| **4°C** | | | | | | |
| 4 weeks | 107.0 | 113.0 | Clear, colourless | No significant degradation was found | | |
| 10 weeks | | | Clear, colourless | | | |
| 11 months | | | Clear, colourless | | | |

**PD01/22b**

| | Assay methadone μg/ml | | Appearance of solution | Degradation % | | |
|---|---|---|---|---|---|---|
| | | | | | | |
| | U1 | U2 | | | | |
| Initial | | | Clear, colourless | | | |
| **4°C** | | | | | | |
| 4 weeks | 114.2 | 114.9 | Clear, colourless | No significant degradation was found | | |
| 10 weeks | | | Clear, colourless | | | |
| 11 months | | | Clear, colourless | | | |

**PD01/36**

| | PD01/36a | | PD01/36b | | PD01/36c | | /PD01/36d | |
|---|---|---|---|---|---|---|---|---|
| | **mg unit$^{-1}$** | **% w/w** | **mg unit$^{-1}$** | **% w/w** | **mg unit$^{-1}$** | **% w/w** | **mg unit$^{-1}$** | **% w/w** |
| Methadone HCl | 60.0 | 13.53 | 60.0 | 12.99 | 10.0 | 2.53 | 10.0 | 2.42 |
| Blackcurrant flavour | 2.2 | 0.50 | 2.2 | 0.48 | 2.0 | 0.51 | 2.0 | 0.48 |
| Na Saccharin | 1.1 | 0.25 | | | 1.0 | 0.25 | | |
| Ethanol, anhydrous | 90.0 | 20.29 | 90.0 | 19.48 | 80.0 | 20.22 | 80.0 | 19.40 |
| Propylene glycol | 110.0 | 24.80 | | | 100.0 | 25.28 | | |
| Glycerol | | | 115.0 | 24.89 | | | 105.0 | 25.46 |
| Water | 180.3 | 40.64 | 194.8 | 42.16 | 202.6 | 51.21 | 215.4 | 52.23 |
| | 443.6 | 100.01 | 462.0 | 100.00 | 395.6 | 100.00 | 412.4 | 99.99 |

After standing at room temperature for 8 days prior to testing all the propylene glycol formulations had gained weight. Additional peaks were found in the chromatograms which were also observed identically in the placebos. These were therefore concluded to be from the excipients and are not degradents and thus were not reported.

**PD0I/36a**

| PD01/36a | | Methadone (mg/dose) | Delivered dose (mg) | Delivered dose as % of fill | Degradation | Ethanol Content %w/w |
|---|---|---|---|---|---|---|
| | | | | | | |
| Initial | (n=3) | 58.1 | ND | | | |
| | | | | | | |
| **5°C** | | | | | | |
| 1 month | | N/A | N/A | N/A | N/A | |
| 2 month | (n=1) | 59.2 | 374.7 | 95.1 | No significant degradation | |
| 6 month | (n=2) | 55.8 | 371.5 | 94.0 | No significant degradation | |
| | | | | | | |
| **25°C/60%RH** | | | | | | |
| 1 month | (n=2) | 57.1 | 359.9 | 91.0 | No significant degradation | 21.0 |
| 2 month | (n=3) | 58.9 | 372.2 | 94.8 | No significant degradation | 17.8 |
| 6 month | (n=2) | 59.6 | 377.8 | 94.6 | No significant degradation | |
| | | | | | | |
| **30°C/65%RH** | | | | | | |
| 1 month | (n=3) | 59.2 | 372.3 | 95.3 | No significant degradation | 20.7 |
| 2 month | (n=2) | 59.3 | 363.7 | 91.1 | No significant degradation | 19.0 |
| 6 month | (n=2) | 60.6 | 369.5 | 94.3 | No significant degradation | |
| | | | | | | |
| **40°C/75%RH** | | | | | | |
| 1 month | (n=3) | 59.7 | 372.6 | 94.9 | No significant degradation | 20.2 |
| 2 month | (n=3) | 57.6 | 363.5 | 93.8 | No significant degradation | 17.3 |
| 6 month | (n=2) | 56.8 | 368.0 | 93.5 | No significant degradation | |

**PD01/36b**

| PD01/36b | | Methadone (mg/dose) | Delivered dose (mg) | Delivered dose as % of fill | Degradation | Ethanol Content %w/w |
|---|---|---|---|---|---|---|
| | | | | | | |

(continued)

| PD01/36b | | Methadone (mg/dose) | Delivered dose (mg) | Delivered dose as % of fill | Degradation | Ethanol Content %w/w |
|---|---|---|---|---|---|---|
| Initial | (n=3) | 58.7 | 386.4 | 92.5 | | |
| | | | | | | |
| **5°C** | | | | | | |
| 1 month | | N/A | N/A | N/A | N/A | |
| 2 month | (n=3) | 56.6 | 385.4 | 93.5 | No significant degradation | |
| 6 month | (n=2) | 56.8 | 391.3 | 93.2 | No significant degradation | |
| | | | | | | |
| **25°C/60%RH** | | | | | | |
| 1 month | (n=2) | 57.3 | 389.6 | 94.1 | No significant degradation | 19.7 |
| 2 month | (n=3) | 57.6 | 378.3 | 92.0 | No significant degradation | 20.4 |
| 6 month | (n=2) | 57.2 | 394.8 | 93.8 | No significant degradation | |
| | | | | | | |
| **30°C/65%RH** | | | | | | |
| 1 month | (n=3) | 60.1 | 398.3 | 95.1 | No significant degradation | 19.0 |
| 2 month | (n=1) | 59.1 | 392.4 | 93.2 | No significant degradation | 19.9 |
| 6 month | (n=2) | 57.0 | 387.9 | 93.9 | No significant degradation | |
| | | | | | | |
| **40°C/75%RH** | | | | | | |
| 1 month | (n=3) | 59.7 | 390.9 | 94.3 | No significant degradation | 19.5 |
| 2 month | (n=1) | 58.4 | 386.9 | 94.1 | No significant degradation | 19.9 |
| 6 month | (n=2) | 57.7 | 388.8 | 93.5 | No significant degradation | |

**PD01/36c**

| PD01/36c | | Methadone (mg/dose) | Delivered dose (mg) | Delivered dose as % of fill | Degradation | Ethanol Content %w/w |
|---|---|---|---|---|---|---|
| | | | | | | |
| Initial | (n=3) | 9.7 | 369.1 | 94.3 | | |

(continued)

| PD01/36c | | Methadone (mg/dose) | Delivered dose (mg) | Delivered dose as % of fill | Degradation | Ethanol Content %w/w |
|---|---|---|---|---|---|---|
| | | | | | | |
| **5°C** | | | | | | |
| 1 month | | N/A | N/A | N/A | N/A | |
| 2 month | (n=3) | 9.5 | 369.5 | 95.0 | No significant degradation | |
| 6 month | (n=2) | 9.0 | 359.9 | 91.9 | No significant degradation | |
| | | | | | | |
| **25°C/60%RH** | | | | | | |
| 1 month | (n=3) | 9.5 | 371.6 | 95.0 | No significant degradation | 18.2 |
| 2 month | (n=3) | 9.8 | 371.2 | 95.2 | No significant degradation | 19.4 |
| 6 month | (n=2) | 9.5 | 367.6 | 93.3 | No significant degradation | |
| | | | | | | |
| **30°C/65%RH** | | | | | | |
| 1 month | (n=2) | 9.6 | 359.6 | 93.0 | No significant degradation | 19.2 |
| 2 month | (n=3) | 9.9 | 370.9 | 95.0 | No significant degradation | 17.0 |
| 6 month | (n=2) | 9.5 | 365.6 | 93.5 | No significant degradation | |
| | | | | | | |
| **40°C/75%RH** | | | | | | |
| 1 month | (n=2) | 10.0 | 375.9 | 95.3 | No significant degradation | 20.6 |
| 2 month | (n=3) | 9.7 | 364.4 | 94.9 | No significant degradation | 18.0 |
| 6 month | (n=2) | 9.6 | 365.4 | 93.2 | No significant degradation | |

**PD01/36d**

| PD01/36d | | Methadone (mg/dose) | Delivered dose (mg) | Delivered dose as % of fill | Degradation | Ethanol Content %w/w |
|---|---|---|---|---|---|---|
| | | | | | | |
| Initial | (n=3) | 10.0 | 391.4 | 95.8 | | |
| | | | | | | |

(continued)

| | | | | | | |
|---|---|---|---|---|---|---|
| **5°C** | | | | | | |
| 1 month | | N/A | N/A | N/A | N/A | |
| 2 month | (n=3) | 9.9 | 385.7 | 94.4 | No significant degradation | |
| 6 month | (n=2) | 9.1 | 377.6 | 93.0 | No significant degradation | |
| | | | | | | |
| **25°C/60%RH** | | | | | | |
| 1 month | (n=2) | 9.8 | 386.3 | 95.6 | No significant degradation | 17.8 |
| 2 month | (n=3) | 10.0 | 388.1 | 95.5 | No significant degradation | 19.2 |
| 6 month | (n=2) | 9.4 | 383.3 | 93.7 | No significant degradation | |
| | | | | | | |
| **30°C/65%RH** | | | | | | |
| 1 month | (n=2) | 10.0 | 393.2 | 95.5 | No significant degradation | 19.7 |
| 2 month | (n=3) | 9.8 | 387.1 | 95.0 | No significant degradation | 17.5 |
| 6 month | (n=2) | 9.6 | 380.7 | 94.5 | No significant degradation | |
| | | | | | | |
| **40°C/75%RH** | | | | | | |
| 1 month | (n=3) | 10.2 | 394.1 | 96.3 | No significant degradation | 19.7 |
| 2 month | (n=2) | 9.7 | 378.4 | 94.5 | No significant degradation | 17.3 |
| 6 month | (n=2) | 9.7 | 384.6 | 93.7 | No significant degradation | |

After three months storage it was observed that some of the 60mg units had leaked and had a white crystalline deposit around the base of the units and plugs. This occurred at all temperatures.

It was also noted that some units stored at 5°C across all formulations and placebos were discoloured pale orange/brown. The stored bulk solutions were still clear and colourless.

At six months pale orange/brown discolouration was noted in a few (2 @ 40°C, 2@30°C & 1@5°C) out of 79 units assayed. Some continuing evidence of poor sealing is shown by high weight loss displayed by some units.

**PD01/049 & 51**

| | **PD01/049** | | **PD01/051** | |
|---|---|---|---|---|
| | g | % w/w | g | % w/w |
| **Methadone HCl** | 15.01 | 14.0 | 2.54 | 2.5 |
| **Ethanol, anhydrous** | 20.08 | 18.8 | 20.21 | 19.7 |

(continued)

| | PD01/049 | | PD01/051 | |
|---|---|---|---|---|
| | g | % w/w | g | % w/w |
| **Glycerol** | 25.00 | 23.4 | 25.08 | 24.4 |
| **Purified water** | 46.96 | 43.9 | 54.98 | 53.5 |
| **TOTAL** | 107.05 | 100.1 | 102.81 | 100.1 |

| **PD01/049** | | **Methadone (mg/dose)** | **Delivered dose (mg)** | **Delivered dose as % of fill** | **Degradation** | **Ethanol Content %w/w** |
|---|---|---|---|---|---|---|
| | | | | | | |
| Initial | n=5 | 55.8 | 390.6 | 93.8 | No significant degradation | 17.7 |
| | | | | | | |
| **5°C** | | | | | | |
| 1 month | n=3 | 55.5 | 394.0 | 96.2 | No significant degradation | 16.9 |
| 2 month | n=4 | 56.7 | 393.8 | 92.6 | No significant degradation | 17.3 |
| 3 month | n=4 | 58.1 | 397.4 | 94.4 | No significant degradation | |
| 6 month | n=3 | 52.1 | 390.4 | 93.4 | No significant degradation | 19.3 |
| | | | | | | |
| **25°C/60%RH** | | | | | | |
| 1 month | n=4 | 56.2 | 389.6 | 93.1 | No significant degradation | 17.5 |
| 2 month | n=4 | 55.9 | 389.6 | 94.0 | No significant degradation | 17.8 |
| 3 month | n=4 | 58.1 | 392.2 | 94.8 | No significant degradation | |
| 6 month | n=4 | 53.0 | 399.2 | 94.8 | No significant degradation | 18.4 |
| | | | | | | |
| **40°C/75%RH** | | | | | | |
| 1 month | n=4 | 55.9 | 387.4 | 95.2 | No significant degradation | 17.9 |
| 2 month | n=4 | 57.7 | 395.9 | 96.3 | No significant degradation | 18.0 |
| 3 month | n=4 | 57.8 | 391.0 | 94.0 | No significant degradation | |

(continued)

| | | | | | | |
|---|---|---|---|---|---|---|
| **40°C/75%RH** | | | | | | |
| 6 month | n=4 | 51.8 | 393.3 | 90.7 | No significant degradation | 18.9 |

| **PD01/051** | | **Methadone (mg/dose)** | **Delivered dose (mg)** | **Delivered dose as % of fill** | **Degradation** | **Ethanol Content %w/w** |
|---|---|---|---|---|---|---|
| | | | | | | |
| Initial | n=5 | 9.6 | 386.3 | 95.7 | No significant degradation | 19.3 |
| | | | | | | |
| **5°C** | | | | | | |
| 1 month | n=3 | 9.8 | 390.0 | 94.5 | No significant degradation | 17.5 |
| 2 month | n=4 | 9.6 | 391.5 | 95.9 | No significant degradation | 18.7 |
| 3 month | n=4 | 10.0 | 400.2 | 96.9 | No significant degradation | |
| 6 month | n=4 | 9.1 | 397.9 | 97.1 | No significant degradation | 16.8 |
| | | | | | | |
| **25°C/60%RH** | | | | | | |
| 1 month | n=3 | 9.3 | 393.7 | 93.3 | No significant degradation | 18.6 |
| 2 month | n=4 | 9.7 | 388.1 | 94.9 | No significant degradation | 19.2 |
| 3 month | n=4 | 9.8 | 384.6 | 93.7 | No significant degradation | |
| 6 month | n=4 | 9.1 | 392.7 | 93.5 | No significant degradation | 18.9 |
| | | | | | | |
| **40°C/75%RH** | | | | | | |
| 1 month | n=3 | 9.7 | 387.0 | 95.1 | No significant degradation | 18.5 |
| 2 month | n=4 | 9.7 | 393.0 | 95.2 | No significant degradation | 18.3 |
| 3 month | n=2 | 9.9 | 386.5 | 94.1 | No significant degradation | |
| 6 month | n=4 | | 398.1 | 97.7 | | 18.6 |

All solutions were clear and colourless

**Phase I Clinical trial Supplies 08-212, 08-213 & 08-214;**

| | 08-212 | 08-213 | 08-214 | Batch ESN |
|---|---|---|---|---|
| | 10mg | 20mg | 30mg | |
| Methadone HCl | 27.0g | 54.0g | 81.0g | 4175 |
| Ethanol, anhydrous | 205.0g | 205.0g | 205.0g | 4163 (plus 35.5g 4180 in 08-214) |
| Glycerol | 250.0g | 250.0g | 250.0g | 4162 |
| Water | 538.0g | 516.0g | 486.0g | 4157 |
| | 1020g | 1025g | 1022g | |
| P = Pouched, U = Unpouched. | | | | |

**08-212, 10mg**

| | Description | Methadone HCl (mg/ml) | Degradation | Uniformity of Content | Mean Methadone HCl (mg/dose) | Uniformity of Mass | Mean Delivered dose (mg) | Ethanol (%w/w) |
|---|---|---|---|---|---|---|---|---|
| **Specification** | | **24.3-29.7** | | | **8.5-11.5** | | | **18.0-22.0** |
| | | | | | | | | |
| **Initial** | Conforms | **28.1** | None | Conforms | **9.9** | Conforms | **375.5** | **18.7** |
| **5°C** | | | | | | | | |
| *1 Month* | Conforms | **27.6** *(U=27.4; P=27.7)* | None | Conforms | **9.5** *(U=9.4; P=9.6)* | Conforms | **373.7** *(U=369.6; P=377.8)* | **20.04** *(U=20.4; P=20.4)* |
| *3 Months* | Conforms | **26.0** *(U=262; P=25.7)* | None | Conforms | **9.6** *(U=9·6;* P=9.6) | Conforms | **377.6** *(U=387.8; P=367.4)* | **20.4** *(U=20.8; P=20.0)* |
| *6 Months* | Conforms | **27.5** *(U=27.2; P=27.8)* | None | Conforms | **9.7** *(U=9.6; P=9.7)* | Conforms | **374.8** *(U=373.4; P=376.2)* | **19.7** *(U=19.4; P=19.9)* |
| *9 Months* | Conforms | **27.2** (U=27.1; P=27.3) | None | Conforms | **9.4** (U=9.4; P=9.4) | Conforms | **362.6** (U=363.0; P=362.2) | **19.7** (U=19.8; P=19.5) |
| *16 months* | Conforms | **28.5** | None | N/A | **N/A** | Conforms | **378.8** | **18.4** |
| **25°C/60% RH** | | | | | | | | |
| *1 Month* | Conforms | **27.1** *(U=27.1; P=27.1)* | None | Conforms | **9.4** *(U=9.4; P=9.3)* | Conforms | **372.1** *(U=372.8; P=371.5)* | **19.9** *(U=19.9; P=19.9)* |
| *3 Months* | Conforms | **26.8** *(U=27.0; P=266)* | None | Conforms | **9.3** *(U=9.4; P=9.3)* | Conforms | **382.0** *(U=381.1; P=382.8)* | **20.7** *(U=20.8; P=20.6)* |
| *6 Months* | Conforms | **27.6** *(U=27.3; P=27.8)* | None | Conforms | **9.5** *(U=9.5; P=9.4)* | Conforms | **372.0** *(U=373.0; P=371.0)* | **20.0** *(U=20.0; P=20.0)* |
| *9 Months* | Conforms | **27.6** (U=27.3; P=27.8) | None | Conforms | **10.1** (U=10.0; P=10.1) | Conforms | **385.3** (U=384.7; P=385.8) | **20.5** (U=20.7; P=20.3) |
| *16 Months* | Conforms | **27.7** (U=27.6; P=27.8) | None | N/A | **N/A** | Conforms | **376.1** (U=378.2; P=374.1) | **18.3** (U=18.0; P=18.6) |

**08-212, 10mg**

| | Description | Methadone HCl (mg/ml) | Degradation | Uniformity of Content | Mean Methadone HCl (mg/dose) | Uniformity of Mass | Mean Delivered dose (mg) | Ethanol (%w/w) |
|---|---|---|---|---|---|---|---|---|
| **Specification** | | **24.3-29.7** | | | **8.5-11.5** | | | **18.0-22.0** |
| | | | | | | | | |
| **30°C/65% RH** | | | | | | | | |
| *1 Month* | Conforms | **27.5** *(U=27.3; P=27.6)* | None | Conforms | **9.3** *(U=9.2; P=9.3)* | Conforms | **363.2** *(U=362.4; P=363.9)* | **19.8** *(U=19.6; P=19.9)* |
| *3 Months* | Conforms | **26.6** *(U=27.0; P=262)* | None | Conforms | **9.4** *(U=9.4; P=9.4)* | Conforms | **386.0** *(U=386.0; P=385.9)* | 20.7 *(U=20.7, P=20.7)* |
| *6 Months* | Conforms | **27.5** *(U=27.6; P=27.4)* | None | Conforms | **9.7** *(U=9.6; P=9.8)* | Conforms | **376.6** *(U=372.5; P=380.6)* | **20.0** *(U=20.4; P=19.5)* |
| *9 Months* | Conforms | **26.9** (U=26.8; P=26.9) | None | Conforms | **10.1** (U=10.1; P=10.1) | Conforms | **382.3** (U=385.7; P=378.9) | **20.6** (U=21.1; P=20.1) |
| *16 Months* | Conforms | **28.3** (U=28.5; P=28.0) | None | N/A | **N/A** | Conforms | **361.8** (U=355.6; P=368.0) | **18.6** (U=18.8; P=18.4) |
| | | | | | | | | |
| **40°C/75% RH** | | | | | | | | |
| *1 Month* | Conforms | **27.4** *(U=27.3; P=27.5)* | None | Conforms | **9.3** *(U=9.4; P=9.2)* | Conforms | **377.0** *(U=386.1; P=367.8)* | **20.6** *(U=20.9; P=20.3)* |
| *3 Months* | Conforms | **27.0** *(U=268; P=27.1)* | None | Conforms | **9.7** *(U=9.6; P=9.9)* | Conforms | **388.7** *(U=387.3; P=390.2)* | **21.1** *(U=20.9; P=21.7)* |
| *6 Months* | Conforms | **27.6** *(U=27.6; P=27.5)* | None | Conforms | **9.7** *(U=9.8; P=9.6)* | Conforms | **374.2** *(U=375.8; P=372.7)* | **20.1** *(U=18.9; P=20.2)* |

All solutions were clear and colourless.

**08-213, 20mg**

| | Description | Methadone HCl (mg/ml) | Degradation | Uniformity of Content | Mean Methadone HCl (mg/dose) | Uniformity of Mass | Mean Delivered dose (mg) | Ethanol (%w/w) |
|---|---|---|---|---|---|---|---|---|
| **Specification** | | **48.6-59.4** | | | **17.0-23.0** | | | **18.0-22.0** |
| | | | | | | | | |
| **Initial** | Conforms | **54.8** | None | Conforms | **19.8** | Conforms | **381.2** | **18.4** |
| **5°C** | | | | | | | | |
| *1 Month* | Conforms | **55.1** *(U=55.1; P=55.0)* | None | Conforms | **19.0** *(U=18.8; P=19.1)* | Conforms | **379.9** *(U=375.9; P=384.0)* | **19.6** *(U=19.5; P=19.7)* |
| *3 Months* | Conforms | **53.4** *(U=52.9; P=53.8)* | None | Conforms | **19.0** *(U=18.7; P=19.3)* | Conforms | **380.8** *(U=372.5; P=384.1)* | **20.6** *(U=20.9; P=20.3)* |
| *6 Months* | Conforms | **52.8** *(U=53.2; P=52.4)* | None | Conforms | **19.3** *(U=19.0; P=19.6)* | Conforms | **382.0** *(U=378.4; P=385.5)* | **20.1** *(U=20.1; P=20.0)* |
| *9 Months* | Conforms | **55.1** (U=55.7; P=54.4) | None | Conforms | **18.6** (U=18.8; P=18.4) | Conforms | **359.5** (U=365.2; P=353.7) | **19.8** (U=19.8; P=19.8) |
| *16 Months* | Conforms | **54.7** | None | N/A | **N/A** | Conforms | **368.1** | **18.1** |
| **25°C/60% RH** | | | | | | | | |
| *1 Month* | Conforms | **53.2** *(U=53.0; P=53.4)* | None | Conforms | **18.7** *(U=18.9; P=18.5)* | Conforms | **372.7** *(U=369.5; P=375.8)* | **19.4** *(U=19.3; P=19.4)* |
| *3 Months* | Conforms | **53.7** *(U=54.0; P=53.3)* | None | Conforms | **19.6** *(U=19.8; P=19.5)* | Conforms | **386.9** *(U=388.7; P=385.1)* | **20.5** *(U=20.7; P=20.2)* |
| *6 Months* | Conforms | **52.9** *(U=53.0; P=52.8)* | None | Conforms | **19.4** *(U=19.5; P=19.3)* | Conforms | **383.3** *(U=384.0; P=382.6)* | **20.1** *(U=20.3; P=19.9)* |
| *9 Months* | Conforms | **54.4** (U=54.7; P=54.0) | None | Conforms | **20.0** (U=19.9; P=20.0) | Conforms | **384.8** (U=382.7; P=386.8) | **20.5** (U=20.4; P=20.6) |
| *16 months* | Conforms | **56.0** (U=56.0; P=56.0) | None | N/A | **N/A** | Conforms | **372.7** (U=366.2; P=379.2) | **18.5** (U=18.9; P=18.1) |

**08-213, 20mg**

| | Description | Methadone HCl (mg/ml) | Degradation | Uniformity of Content | Mean Methadone HCl (mg/dose) | Uniformity of Mass | Mean Delivered dose (mg) | Ethanol (%w/w) |
|---|---|---|---|---|---|---|---|---|
| **Specification** | | **48.6-59.4** | | | **17.0-23.0** | | | **18.0-22.0** |
| | | | | | | | | |
| **30°C/65% RH** | | | | | | | | |
| *1 Month* | Conforms | **53.6** *(U=53.5; P=53.6)* | None | Conforms | **18.5** *(U=18.2; P=18.7)* | Conforms | **369.6** *(U=364.0; P=375.2)* | **19.5** *(U=19.6; P=19.3)* |
| *3 Months* | Conforms | **52.8** *(U=52.8; P=52.7)* | None | Conforms | **19.8** *(U=19.5; P=20.1)* | Conforms | **387.6** *(U=383.9; P=391.2)* | **21.1** *(U=21.3; P=20.9)* |
| *6 Months* | Conforms | **53.7** *(U=53.7; P=53.6)* | None | Conforms | **19.9** *(U=19.8; P=20.0)* | Conforms | **390.6** *(U=390.2; P=391.1)* | **20.2** *(U=19.9; P=20.0)* |
| *9 Months* | Conforms | **54.4** (U=54.7; P=54.0) | None | Conforms | **20.0** (U=19.9; P=20.0) | Conforms | **384.8** (U=382.7; P=386.8) | **20.5** (U=20.4; P=20.6) |
| *16 Months* | Conforms | **59.4** (U=57.9; P=60.9) | None | N/A | **N/A** | Conforms | **375.2** (U=381.4; P=369.0) | **17.8** (U=17.5; P=18.1) |
| | | | | | | | | |
| **40°C/75% RH** | | | | | | | | |
| *1 Month* | Conforms | **54.8** *(U=54.5; P=55.1)* | None | Conforms | **18.6** *(U=18.5; P=18.7)* | Conforms | **371.3** *(U=369.9; P=372.8)* | **19.4** *(U=19.4; P=NR)* |
| *3 Months* | Conforms | **54.0** *(U=53.9; P=54.1)* | None | Conforms | **19.9** *(U=19.6; P=20.2)* | Conforms | **388.4** *(U=387.8; P=388.9)* | **20.7** *(U=20.9; P=20.4)* |
| *6 Months* | Conforms | **53.2** *(U=53.0; P=53.4)* | None | Conforms | **19.9** *(U=19.8; P=20.0)* | Conforms | **388.4** *(U=389.0; P=387.7)* | **19.7** *(U=20.3; P=19.2)* |

All solutions were clear and colourless.

**08-214, 30mg**

| | Description | Methadone HCl (mg/ml) | Degradation | Uniformity of Content | Mean Methadone HCl (mg/dose) | Uniformity of Mass | Mean Delivered dose (mg) | Ethanol (%w/w) |
|---|---|---|---|---|---|---|---|---|
| **Specification** | | **72.9-89.1** | | | **25.5-34.5** | | | **18.0-22.0** |
| | | | | | | | | |
| **Initial** | Conforms | **81.7** | None | Conforms | **29.3** | Conforms | **377.7** | **19.2** |
| | | | | | | | | |
| **5°C** | | | | | | | | |
| *1 Month* | Conforms | **80.2** *(U=80.6; P=79.8)* | None | Conforms | **27.9** *(U=27.8; P=28.0)* | Conforms | **361.3** *(U=359.8; P=362.8)* | **19.8** *(U=19.3; P=20.3)* |
| *3 Months* | Conforms | **78.4** *(U=76.7; P=80.1)* | None | Conforms | **26.8** *(U=269; P=26.6)* | Conforms | **378.3** *(U=376.6; P=380.1)* | **21.2** *(U=21.4; P=20.9)* |
| *6 Months* | Conforms | **78.6** *(U=78.7, P=78.5)* | None | Conforms | **27.7** *(U=27.7; P=27.8)* | Conforms | **369.0** *(U=367.6; P=370.5)* | **20.4** *(U=20.6; P=20.3)* |
| *9 Months* | Conforms | **80.2** (U=79.0; P=81.4) | None | Conforms | **27.7** (U=27.7; P=27.6) | Conforms | **360.0** (U=368.6; P=351.4) | **20.3** (U=20.2; P=20.6) |
| | | | | | | | | |
| **25°C/60% RH** | | | | | | | | |
| *1 Month* | Conforms | **78.7** *(U=78.5; P=78.8)* | None | Conforms | **28.1** *(U=27.5; P=28.7)* | Conforms | **366.2** *(U=361.9; P=370.4)* | **18.7** *(U=17.9; P=19.5)* |
| *3 Months* | Conforms | **80.6** *(U=80.7; P=80.4)* | None | Conforms | **27.4** *(U=27.1;* P=27.7) | Conforms | **378.8** *(U=374.6; P=383.1)* | **20.9** *(U=20.9; P=20.8)* |
| *6 Months* | Conforms | **79.0** *(U=79.0; P=78.9)* | None | Conforms | **29.2** *(U=29.5; P=29.0)* | N/A | N/A | **20.5** *(U=20.8; P=20.2)* |
| *9 Months* | Conforms | **82.4** (U=82.2; P=82.5) | None | Conforms | **29.1** (U=28.9; P=29.3) | Conforms | **377.0** (U=374.4; P=379.6) | **20.7** (U=20.8; P=20.6) |

**08-214, 30mg**

| | Description | Methadone HCl (mg/ml) | Degradation | Uniformity of Content | Mean Methadone HCl (mg/dose) | Uniformity of Mass | Mean Delivered dose (mg) | Ethanol (%w/w) |
|---|---|---|---|---|---|---|---|---|
| **Specification** | | **72.9-89.1** | | | **25.5-34.5** | | | **18.0-22.0** |
| | | | | | | | | |
| **30°C/65% RH** | | | | | | | | |
| *1 Month* | Conforms | **79.4** *(U=80.4; P=78.4)* | None | Conforms | **28.0** *(U=28.1; P=27.8)* | Conforms | **364.0** *(U=366.2; P=361.7)* | **19.6** *(U=19.7; P=19.5)* |
| *3 Months* | Conforms | **80.2** *(U=79.9; P=80.4)* | None | Conforms | **28.5** *(U=28.2; P=28.7)* | Conforms | **387.3** *(U=384.7; P=390.0)* | **20.5** *(U=20.7; P=20.3)* |
| *6 Months* | Conforms | **79.2** *(U=79.2; P=79.1)* | None | Conforms | **29.5** *(U=29.4; P=29.5)* | Conforms | **386.9** *(U=389.2; P=384.6)* | **20.4** *(U=20.6; P=20.3)* |
| *9 Months* | Conforms | **80.9** (U=81.5; P=80.3) | None | Conforms | **29.4** (U=29.7; P=29.2) | Conforms | **383.6** (U=385.4; P=381.8) | **20.7** (U=21.0; P=20.4) |
| | | | | | | | | |
| **40°C/75% RH** | | | | | | | | |
| *1 Month* | Conforms | **80.3** *(U=80.4; P=80.1)* | None | Conforms | **28.0** *(U=28.0; P=27.9)* | Conforms | **362.5** *(U=364.4; P=360.5)* | **19.6** *(U=19.6; P=NR)* |
| *3 Months* | Conforms | **81.2** *(U=81.9; P=80.5)* | None | Conforms | **28.4** *(U=28.5; P=28.4)* | Conforms | **389.8** *(U=388.9; P=390.7)* | **20.7** *(U=20.6; P=20.7)* |
| *6 Months* | Conforms | **79.9** *(U=79.8; P=80.0)* | None | Conforms | **29.5** *(U=29.5; P=29.5)* | Conforms | **389.2** *(U=392.9; P=385.6)* | **20.4** *(U=20.3; P=20.6)* |

All solutions were clear and colourless.

Pharmacokinetic studies

A confidential phase 1, single centre, open label, semi randomized three way crossover trial was carried out to determine the pharmacokinetics of single doses of methadone sublingual spray, and to establish the relative bioavailability with methadone syrup in healthy male subjects.

Objectives:

The primary objectives of this study were to:

- assess the single dose pharmacokinetics of methadone sublingual spray in healthy male subjects;
- determine the bioavailability of methadone sublingual spray relative to methadone syrup in healthy male subjects; and
- determine the dose proportionality between two different doses of methadone sublingual spray

The secondary objectives of this study were to:

- establish the safety, tolerability and taste acceptance of methadone sublingual spray in healthy subj ects

Methodology/study design:

Subjects were required to provide their written informed consent prior to any study related procedures being conducted. Subjects were screened for eligibility within 28 days of first study admission on Day -1. Eligible subjects were required to participate in three study periods, with a washout interval of at least one week between the study periods. For each treatment period, subjects were admitted to the clinical unit the evening prior to dosing. Naltrexone block was administered to subjects at the investigators discretion. Subjects received their three treatments in a semi-randomised way such that the highest dose sublingual spray was only given after each individual subject had first received the lower dose sublingual spray. Subjects were closely monitored in the clinic for at least 24 hours after dosing and returned for 2 outpatient visits for blood sampling. After the last treatment period, subjects returned for a post study follow up visit.

Number of subjects (analysed):

A total of 7 healthy male subjects were enrolled in the study. One subject was withdrawn following the first treatment period (methadone syrup). The replacement subject completed the remaining two treatment periods. Five subjects completed all three treatment periods. Seven (7) subjects entered the study and were included in the safety and pharmacokinetic populations.

Diagnosis and Main Criteria for Inclusion:

Healthy male subjects aged 18-45 and with a BMI within the range 18-29 were eligible for the trial.

Test Product, Dose and Mode of Administration Batch Number(s):

Methadone sublingual spay 10mg/actuation, batch number 08-212 (Treatment A) Methadone sublingual spay 20mg/actuation, batch number 08-213 (Treatment B) Reference Product, Dose and Mode of Administration, Batch Number(s): Biophine syrup (5mg methadone HC1/mL), batch number 100492 (Treatment C)

Duration of Treatment:

The planned study duration was approximately 8 weeks, while the duration of treatment was approximately 3 weeks.

Criteria for Evaluation:

Pharmacokinetic variables -

Blood samples were collected for pharmacokinetic analysis at the following timepoints: Predose and 2, 5, 10,

15, 30, 45, 60 minutes and 1.5, 2, 3, 4, 6, 8, 10, 12, 24, 48 and 72 hours after dosing .The following pharmacokinetic parameters for methadone were calculated by standard non-compartmental methods using WinNonlin Ver 5.0.1: AUC $_{0-t}$, AUC 0-∞, $C_{max}$, $T_{max}$, $\lambda_z$, $t_{1/2}$, CL/F, V/F and F. SPSS Ver 17.0 was used for the statistical analysis.

Statistical Methods:

All statistical analyses were appropriate to the nature and distribution of the data collected. These are detailed in the pharmacokinetic analysis plan.

Pharmacokinetics -

Pharmacokinetic samples from all treatment periods were analysed for methadone concentrations. Statistical analysis was based on data from all treatment periods for all subjects studied. Individual subject profiles and mean profiles of the plasma concentration for methadone by treatment were produced.

The pharmacokinetic parameters AUC $_{0-t}$, AUC $_{0-\infty}$, $C_{max}$, $T_{max}$, $\lambda_z$, $t_{1/2}$, CL/F, V/F and F were listed by treatment, and where appropriate suitable statistical comparisons were made.

Summary of Pharmacokinetic Results and Conclusions:

Summary PK Parameters for methadone by treatment -

| Pharmacokinetic Parameter | Summary Statistic | Treatment A | Treatment B | Treatment C |
|---|---|---|---|---|
| $AUC_{0-72}$ (ng.h/mL) | Mean CV% | 1067.49<br>8.34 | 2147.34<br>8.38 | 1287.72<br>17.61 |
| $AUC_{0-\infty}$ (ng.h/mL) | Mean CV% | 1319.17<br>7.87 | 2752.84<br>12.95 | 1777.47<br>22.60 |
| $C_{max}$ (ng/mL) | Mean CV% | 35.9<br>15.95 | 73.4<br>13.20 | 44.4<br>19.99 |
| $t_{½}$ (hours) | Mean CV% | 29.65<br>9.80 | 31.51<br>13.34 | 37.73<br>19.83 |
| $t_{max}$ (hours) | Median Range | 3.0<br>1.5-6.0 | 4.0<br>0.8-4.0 | 2.0<br>1.5-8.0 |
| V/F (mL) | Mean CV% | 326101<br>13.18 | 331044<br>8.18 | 312280<br>18.24 |
| CL/F (mL/hour) | Mean CV% | 7617.50<br>8.07 | 7364.60<br>12.58 | 5841.57<br>19.89 |

Summary of statistical analysis of bioavailability and dose proportionality -

| Pharmacokinetic Parameter | Summary Statistic | Treatment A/ Treatment C | Treatment B/ Treatment C | Treatment B/ Treatment A |
|---|---|---|---|---|
| $Log_{10}$ AUC $_{0-72}$ | Ratio of means<br>90% CI | 0.845<br>0.733 - 0.973 | 1.747<br>1.483 - 2.058 | 2.011<br>1.841- 2.197 |
| $Log_{10}$ $C_{max}$ | Ratio of means<br>90% CI | 0.825<br>0.650 - 1.048 | 1.689<br>1.392 - 2.050 | 2.050<br>1.757 - 2.392 |
| $T_{max}$ [a] | Z P value | 0.37<br>0.72 | 0<br>1.00 | 0.42<br>0.67 |

(continued)

| Pharmacokinetic Parameter | Summary Statistic | Treatment A/ Treatment C | Treatment B/ Treatment C | Treatment B/ Treatment A |
|---|---|---|---|---|
| $t_{1/2}$ | t P value | 2.47<br>0.03 | 1.78<br>0.11 | -0.89<br>0.39 |

Following sublingual administration of methadone, the rate of absorption as indicated by $t_{max}$ was slower than that for the oral comparator dosage form. Additionally, the $t_{max}$ was longer (4.0h vs 3.0h) for treatment B (20mg methadone sublingual spray) than for treatment A (10mg methadone sublingual spray). The relative bioavailability of treatment A compared to treatment C was 84.5% based on AUC and 82.5% based on $C_{max}$: this increases to about 88% if comparisons are made on paired observations only. The $t_{1/2}$ for the oral formulation was about 20% longer than for treatment A, the sublingual formulation, a difference that was statistically significant. Treatments A and B were shown to be dose proportional.

A comparison of plasma methadone concentration profiles for sublingual and syrup administration of a 10mg dose is shown in Figure 1. Surprisingly, sublingual administration does not lead to an initial spike in blood methadone concentration, in comparison to what has been previously observed with other opioids delivered sublingually (and to some extent in comparison to the oral route as demonstrated by the syrup data).

For most of the PK parameters, the variability as evidenced by CV% was lower following the sublingual route of administration than for that following the oral route of administration, making the sublingual composition of the invention a safer methadone product.

Example 2 - fentanyl formulation

| | **200 μg/dose** | |
|---|---|---|
| | **Weight (g)** | **% (w/w)** |
| **Fentanyl** | 0.134 | 0.06 |
| **Propyl parabens** | 1.000 | 0.42 |
| **Orange oil** | 2.003 | 0.85 |
| **Miglyol** | 232.508 | 98.67 |
| **TOTAL** | 235.645 | 100.00 |

| | **800 μg/dose** | |
|---|---|---|
| | **Weight (g)** | **% (w/w)** |
| **Fentanyl** | 0.541 | 0.23 |
| **Propyl parabens** | 1.000 | 0.42 |
| **Orange oil** | 2.004 | 0.85 |
| **Miglyol** | 232.205 | 98.50 |
| **TOTAL** | 235.750 | 100.00 |

Stability for Fentanyl in miglyol: 200ug/dose

| | | **Fentanyl base (μg/dose)** | **Delivered Dose by Weight (mg)** | **% dose delivered by weight** |
|---|---|---|---|---|
| | | | | |

(continued)

| | | Fentanyl base (μg/dose) | Delivered Dose by Weight (mg) | % dose delivered by weight |
|---|---|---|---|---|
| Initial | | 169 (n=5) | 343.6 (n=5) | 88.7 (n=5) |
| | | | | |
| **5°C** | | | | |
| 1 month | | | | |
| 2 months | | 170.8 (n=5) | 345.6 (n=5) | 89.2 (n=5) |
| 3 months | | 179.0 (n=5) | 348.7 (n=5) | 90.0 (n=5) |
| 6 months | | 175.2 (n=5) | 346.4 (n=5) | 89.4 (n=5) |
| | | | | |
| **25°C/60%RH** | | | | |
| 6 months | | 166.4 (n=5) | 343.0 (n=5) | 88.5 (n=5) |
| | | | | |
| **30°C/65%RH** | | | | |
| 6 months | | 163.4 (n=5) | 332.0 (n=5) | 85.7 (n=5) |
| | | | | |
| **40°C/75%RH** | | | | |
| 1 month | | 171.0 (n=5) | | |
| 2 months | | 161.4 (n=5) | 330.9 (n=5) | 85.4 (n=5) |
| 3 months | | 158.8 (n=5) | 331.8 (n=5) | 85.6 (n=5) |
| 6 months | | 145.8 (n=5) | 313.0 (n=5) | 80.8 (n=5) |

Stability for Fentanyl in miglyol: 800ug/dose

| | | Fentanyl base (μg/dose) | Delivered Dose by Weight (mg) | % dose delivered by weight |
|---|---|---|---|---|
| | | | | |
| Initial | | 662.3 (n=4) | 338.1 (n=4) | 87.3 (n=4) |
| | | | | |
| **5°C** | | | | |
| 1 month | | 733.0 (n=5) | | |
| 2 months | | 680.8 (n=5) | 344.8 (n=5) | 90.0 (n=5) |
| 3 months | | 713.2 (n=5) | 343.9 (n=5) | 88.7 (n=5) |
| 6 months | | 697.8 (n=5) | 342.7 (n=5) | 88.4 (n=5) |
| | | | | |
| **25°C/60%RH** | | | | |
| 6 months | | 637.6 (n=5) | 332.1 (n=5) | 85.7 (n=5) |
| | | | | |

(continued)

| | | | | |
|---|---|---|---|---|
| **30°C/65%RH** | | | | |
| 6 months | | 623.2 (n=5) | 333.2 (n=5) | 86.0 (n=5) |
| | | | | |
| **40°C/75%RH** | | | | |
| 1 month | | 677.4 (n=5) | | |
| 2 months | | 638.6 (n=5) | 333.0 (n=5) | 85.9 (n=5) |
| 3 months | | 631.8 (n=5) | 333.5 (n=5) | 86.1 (n=5) |
| 6 months | | 571.4 (n=5) | 314.6 (n=5) | 81.2 (n=5) |

Miglyol is not compatible with Topas, therefore this formulation had to be used in polypropylene. No degradation of fentanyl was observed at any timepoint or temperature but there was some indication of degradation of the orange flavour.

## Claims

**1.** A pharmaceutical composition for the sublingual delivery of an opioid comprising an opioid and a medium chain length triglyceride wherein the composition is comprised within a container and wherein the material of the container that is in contact with the composition is polypropylene.

**2.** A composition according to claim 1 wherein the opioid is not methadone.

**3.** A composition according to claim 1 wherein said opioid is fentanyl.

**4.** A composition according to any one of claims 1 to 3 wherein the container comprises a delivery device.

**5.** A composition according to claim 4 wherein the delivery device dispenses the composition in a single discharge.

**6.** A composition according to claim 4 or claim 5 wherein the delivery device dispenses the composition as a spray.

**7.** A composition according to claim 6 wherein said spray comprises liquid droplets having a mean diameter of at least about 10 microns, preferably at least about 20 microns, more preferably between about 20 microns and about 200 microns, and most preferably between about 20 microns and about 100 microns.

**8.** A composition according to any one of claims 4 to 7 wherein the delivery device is non-pressurised.

**9.** A composition according to any one of claims 4 to 8 wherein the delivery device comprises seals and/or plungers and wherein the material of said seals and/or plungers that is in contact with the composition is bromobutyl polymer.

**Figure 1**

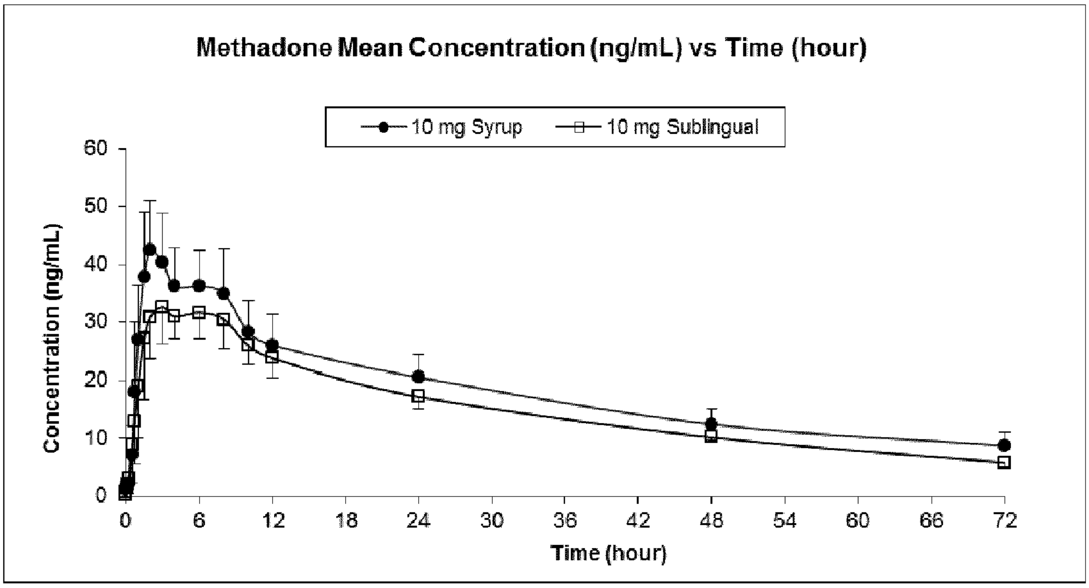
Methadone Mean Concentration (ng/mL) vs Time (hour)
10 mg Syrup
10 mg Sublingual
Concentration (ng/mL)
Time (hour)
0
10
20
30
40
50
60
0
6
12
18
24
30
36
42
48
54
60
66
72

Europäisches Patentamt
European Patent Office
Office européen des brevets

# EUROPEAN SEARCH REPORT

Application Number
EP 13 17 6687

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2007/087431 A2 (INSYS THERAPEUTICS INC [US]; KOTTAYIL S GEORGE [US]; GOSKONDA VENKAT R) 2 August 2007 (2007-08-02)<br>* paragraphs [0091] - [0100], [0136], [0138]; claim 54; examples *<br>----- | 1-9 | INV.<br>A61K9/00<br>A61K9/12<br>A61P25/36<br>A61P25/04<br>A61K31/4468 |
| Y | WO 2009/017837 A2 (INSYS THERAPEUTICS INC [US]; KOTTAYIL S GEORGE [US]; ZHU ZHONGYUAN [US)<br>5 February 2009 (2009-02-05)<br>* claims 35-37,54,59,76; example 5 *<br>----- | 1-9 | |
| Y | WO 2009/070829 A1 (KRIUS PTY LTD [AU]; KELLY ALEXANDER JAMES [AU])<br>11 June 2009 (2009-06-11)<br>* page 3, lines 21-25 *<br>* page 5, line 14 - page 6 *<br>----- | 1-6 | |
| Y | WO 97/49402 A1 (JANSSEN PHARMACEUTICA NV [BE]; VLAMINCK KATHLEEN MARIE JEANNE [BE]; FR) 31 December 1997 (1997-12-31)<br>* claims; examples *<br>-----<br>-/-- | 1,2 | TECHNICAL FIELDS SEARCHED (IPC)<br>A61K<br>A61P |

The present search report has been drawn up for all claims

| Place of search | Date of completion of the search | Examiner |
|---|---|---|
| Munich | 17 September 2013 | Pacreu Largo, Marta |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2

EPO FORM 1503 03.82 (P04C01)

Europäisches Patentamt
European Patent Office
Office européen des brevets

# EUROPEAN SEARCH REPORT

Application Number
EP 13 17 6687

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | European Directorate for the Quality of Medicines (EDQM), Council of Europe: "3.2.2 Plasic containers and closures for pharmaceutical use", European Pharmacopoeia 5.0 , 15 June 2004 (2004-06-15), pages 308-309, XP002667819, Retrieved from the Internet: URL:http://lib.njutcm.edu.cn/yaodian/ep/EP5.0/03_materials_and_containers/3.2.__containers/3.2.2.%20Plastic%20containers%20and%20closures%20for%20pharmaceutical%20use.pdf [retrieved on 2012-01-24] * page 308, right-hand column, paragraph third * ----- | 1 | |
| Y | DONNELLY R F: "Physical compatibility and chemical stability of bupivacaine and hydromorphone in polypropylene syringes", CANADIAN SOCIETY OF HOSPITAL PHARMACY, vol. 57, no. 4, 1 January 2004 (2004-01-01), pages 230-235, XP009155751, CANADIAN SOCIETY OF HOSPITAL PHARMACISTS, OTTAWA ISSN: 0008-4123 * abstract * ----- | 1,2 | TECHNICAL FIELDS SEARCHED (IPC) |
| Y | WO 2009/152949 A1 (POHL BOSKAMP GMBH & CO KG G [DE]; GROTELUESCHEN ROLF [DE]; UECK HENNIN) 23 December 2009 (2009-12-23) * page 1 - page 4; claims * ----- | 1-6,8 | |
| Y | US 2006/062812 A1 (ROSS CALVIN [GB] ET AL) 23 March 2006 (2006-03-23) * paragraphs [0064] - [0065]; claims * ----- -/-- | 1-6,8 | |

The present search report has been drawn up for all claims

2

| Place of search | Date of completion of the search | Examiner |
|---|---|---|
| Munich | 17 September 2013 | Pacreu Largo, Marta |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 13 17 6687

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2008/047163 A1 (PHARMASOL LTD [GB]; BOOLES CLIVE [GB]; O'BRIEN PADRIAC [GB]) 24 April 2008 (2008-04-24) * page 11; claims * ----- | 1-6,8 | |
| Y | WO 2007/007059 A1 (SOSEI R & D LTD [GB]; BOOLES CLIVE [GB]; ROSS CALVIN [GB]) 18 January 2007 (2007-01-18) * page 4; claims * ----- | 1-6,8 | |
| Y,P | WO 2010/122276 A1 (LONDONPHARMA LTD [GB]; BOOLES CLIVE [GB]) 28 October 2010 (2010-10-28) * page 2, lines 14-30; examples B,C * ----- | 1-8 | |

TECHNICAL FIELDS SEARCHED (IPC)

The present search report has been drawn up for all claims

2

| Place of search | Date of completion of the search | Examiner |
|---|---|---|
| Munich | 17 September 2013 | Pacreu Largo, Marta |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 13 17 6687

This annex lists the patent family members relating to the patent documents cited in the above-mentioned European search report.
The members are as contained in the European Patent Office EDP file on
The European Patent Office is in no way liable for these particulars which are merely given for the purpose of information.

17-09-2013

| Patent document cited in search report | Publication date | Patent family member(s) | Publication date |
|---|---|---|---|
| WO 2007087431 A2 | 02-08-2007 | AU 2007208229 A1 | 02-08-2007 |
| | | BR PI0707235 A2 | 26-04-2011 |
| | | CA 2637672 A1 | 02-08-2007 |
| | | CN 101378735 A | 04-03-2009 |
| | | EP 1976521 A2 | 08-10-2008 |
| | | EP 2641595 A1 | 25-09-2013 |
| | | JP 5241514 B2 | 17-07-2013 |
| | | JP 2009528272 A | 06-08-2009 |
| | | JP 2013056928 A | 28-03-2013 |
| | | JP 2013127003 A | 27-06-2013 |
| | | NZ 569949 A | 28-10-2011 |
| | | US 2007261695 A1 | 15-11-2007 |
| | | WO 2007087431 A2 | 02-08-2007 |
| WO 2009017837 A2 | 05-02-2009 | AU 2008282743 A1 | 05-02-2009 |
| | | CA 2698749 A1 | 05-02-2009 |
| | | EP 2180844 A2 | 05-05-2010 |
| | | US 2009176834 A1 | 09-07-2009 |
| | | WO 2009017837 A2 | 05-02-2009 |
| WO 2009070829 A1 | 11-06-2009 | NONE | |
| WO 9749402 A1 | 31-12-1997 | AU 712333 B2 | 04-11-1999 |
| | | AU 3341897 A | 14-01-1998 |
| | | JP 2000512649 A | 26-09-2000 |
| | | US 6113937 A | 05-09-2000 |
| | | WO 9749402 A1 | 31-12-1997 |
| | | ZA 9705703 A | 28-12-1998 |
| WO 2009152949 A1 | 23-12-2009 | AU 2009259709 A1 | 23-12-2009 |
| | | CA 2727377 A1 | 23-12-2009 |
| | | DE 202008008079 U1 | 04-09-2008 |
| | | EP 2288326 A1 | 02-03-2011 |
| | | RU 2011101404 A | 27-07-2012 |
| | | US 2011098366 A1 | 28-04-2011 |
| | | WO 2009152949 A1 | 23-12-2009 |
| US 2006062812 A1 | 23-03-2006 | NONE | |
| WO 2008047163 A1 | 24-04-2008 | AU 2007311621 A1 | 24-04-2008 |
| | | CA 2666581 A1 | 24-04-2008 |
| | | CN 101573116 A | 04-11-2009 |
| | | EP 2081574 A1 | 29-07-2009 |
| | | GB 2456434 A | 22-07-2009 |
| | | WO 2008047163 A1 | 24-04-2008 |
| | | ZA 200902123 A | 28-04-2010 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 13 17 6687

This annex lists the patent family members relating to the patent documents cited in the above-mentioned European search report.
The members are as contained in the European Patent Office EDP file on
The European Patent Office is in no way liable for these particulars which are merely given for the purpose of information.

17-09-2013

| Patent document cited in search report | Publication date | Patent family member(s) | Publication date |
|---|---|---|---|
| WO 2007007059 A1 | 18-01-2007 | AT 490762 T | 15-12-2010 |
| | | AU 2006268052 A1 | 18-01-2007 |
| | | BR PI0612771 A2 | 30-11-2010 |
| | | CA 2614420 A1 | 18-01-2007 |
| | | CN 101247788 A | 20-08-2008 |
| | | EP 1904032 A1 | 02-04-2008 |
| | | JP 2009500387 A | 08-01-2009 |
| | | KR 20080044232 A | 20-05-2008 |
| | | WO 2007007059 A1 | 18-01-2007 |
| | | ZA 200800147 A | 29-07-2009 |
| WO 2010122276 A1 | 28-10-2010 | NONE | |

EPO FORM P0459

For more details about this annex : see Official Journal of the European Patent Office, No. 12/82